# EUROPEAN PATENT APPLICATION

(11) **EP 3 900 734 A1**
(43) Date of publication of application: **27.10.2021**
(21) Application number: 19897547.6
(22) Date of filing: 23.12.2019
(51) Int. Cl.: A61K 38/28, A61K 38/26, A61K 47/68, A61K 47/60, A61P 3/10, C07K 14/62, C07K 14/605

(54) **PHARMACEUTICAL COMPOSITION CONTAINING INSULIN AND GLUCAGON**

(30) Priority: 21.12.2018 KR 20180167798
(71) Applicant: Hanmi Pharm. Co., Ltd., Hwaseong-si, Gyeonggi-do 18536 (KR)
(72) Inventor: KIM, Jung Kuk, Hwaseong-si, Gyeonggi-do 18469 (KR); LEE, Jong Suk, Hwaseong-si, Gyeonggi-do 18469 (KR); DONG, Joo Young, Hwaseong-si, Gyeonggi-do 18469 (KR); LEE, A Ram, Hwaseong-si, Gyeonggi-do 18469 (KR)
(74) Representative: Mewburn Ellis LLP
(86) International application number: PCT/KR2019/018320
(87) International publication number: WO 2020/130751

(57) **Abstract**

Provided are a composition and a complex formulation, each including insulin or a long-acting conjugate thereof and glucagon or a long-acting conjugate thereof.

## Description

### [Technical Field]

The present invention relates to a composition including insulin and glucagon, and a complex formulation including the same.

### [Background Art]

Glucagon is produced and secreted from the pancreas when blood glucose levels begin to fall due to various causes such as drug treatment, diseases, hormone or enzyme deficiency, *etc.* The secreted glucagon acts on the liver to break down glycogen, inducing release of glucose and eventually causing blood glucose levels to return to normal.

Like glucagon, insulin, which is a kind of hormone produced in the pancreas, is a hormone secreted from the pancreas, and has a role in maintaining blood glucose levels at an appropriate level when the blood glucose levels increase. Deficiency, lack, or malfunction of insulin may cause various diseases. Therefore, administration of insulin is known as a key therapy for the treatment of insulin-related diseases, including diabetes, in which blood glucose levels are high.

The glucagon and insulin are widely known to exert opposite actions, i.e., antagonistic actions on blood glucose, and have been used as therapeutic agents for different diseases. In particular, Korean Patent Publication No. 10-2017-0023066 discloses a method of treating diabetes by supplementing insulin while antagonizing glucagon actions by blocking glucagon receptors with an antagonistic antigen protein.

In other words, since insulin and glucagon have opposite roles in the body, there is no known drug therapy for administering them together.

Meanwhile, when insulin, which is a therapeutic agent for various insulin-related diseases, is administered to a patient, there is a risk of side effects such as weight gain, overdose, and hypoglycemia.

Accordingly, there is a need to develop a drug capable of improving these side effects while maintaining the efficacy of insulin administration.

### [Disclosure]

### [Technical Problem]

An object of the present invention is to provide a pharmaceutical composition for preventing or treating an insulin-related disease, the pharmaceutical composition including insulin and glucagon.

Another object of the present invention is to provide a pharmaceutical composition for preventing or treating hypoglycemia, the pharmaceutical composition including insulin and glucagon.

Still another object of the present invention is to provide a composition for improving side effects of insulin, the composition including insulin and glucagon.

Still another object of the present invention is to provide a complex formulation for improving hypoglycemia of a patient with an insulin-related disease, the complex formulation including insulin and glucagon.

Still another object of the present invention is to provide use of the composition in the preparation of a medicament.

Still another object of the present invention is to provide a method of preventing or treating an insulin-related disease, the method including the step of administering the composition or the complex formulation to an individual.

Still another object of the present invention is to provide a method of improving side effects of insulin, the method including the step of administering the composition or the complex formulation to an individual.

### [Technical Solution]

To achieve the present invention, an aspect provides a pharmaceutical composition for preventing or treating an insulin-related disease, the pharmaceutical composition including insulin and glucagon.

The composition according to one specific embodiment is characterized in that the insulin is linked to a biocompatible material capable of increasing *in vivo* half-life thereof to be in the form of a long-acting conjugate.

The composition according to the above specific embodiment is characterized in that the glucagon is linked to a biocompatible material capable of increasing *in vivo* half-life thereof to be in the form of a long-acting conjugate.

The composition according to any one of the above specific embodiments is characterized in that the insulin is linked to a biocompatible material capable of increasing *in vivo* half-life thereof to be in the form of a long-acting conjugate, and the glucagon is linked to a biocompatible material capable of increasing *in vivo* half-life thereof to be in the form of a long-acting conjugate.

The composition according to any one of the above specific embodiments is characterized in that the insulin-related disease is selected from the group consisting of an insulin-resistant disease, diabetes, hyperglycemia, and obesity.

The composition according to any one of the above specific embodiments is characterized in that the composition alleviates hypoglycemia, which is a side effect of insulin, and suppresses weight gain.

The composition according to any one of the above specific embodiments is characterized in that the composition includes the insulin or the long-acting conjugate thereof and the glucagon or the long-acting conjugate thereof at a weight ratio of 1:1 to 100:1.

The composition according to any one of the above specific embodiments is characterized in that the glucagon is a native glucagon, or a glucagon analog obtained by alteration selected from the group consisting of substitution, addition, deletion, modification, and a combination thereof in one or more amino acids of the native glucagon.

The composition according to any one of the above specific embodiments is characterized in that the glucagon analog includes an amino acid sequence of the following General Formula 1: X1–X2–QGTF–X7–SD–X10–S–X12–X13–X14–X15–X16–X17–X18–X19– 
X20–X21–F–X23–X24–W–L–X27–X28–T–X30 (General Formula 1, SEQ ID
NO: 46)
 in General Formula 1, X1 is histidine (H), desamino-histidyl, dimethyl-histidyl (*N*-dimethyl-histidyl), *beta*-hydroxy imidazopropionyl, 4-imidazoacetyl, *beta*-carboxy imidazopropionyl, tryptophan (W), or tyrosine (Y), or is absent;
X2 is *alpha*-methyl-glutamic acid (a-methyl-glutamic acid), Aib (aminoisobutyric acid), D-alanine, glycine (G), Sar (*N*-methylglycine), serine (S), or D-serine;
X7 is threonine (T), valine (V), or cysteine (C);
X10 is tyrosine (Y) or cysteine (C);
X12 is lysine (K) or cysteine (C);
X13 is tyrosine (Y) or cysteine (C);
X14 is leucine (L) or cysteine (C);
X15 is aspartic acid (D), glutamic acid (E), or cysteine (C);
X16 is glutamic acid (E), aspartic acid (D), serine (S), *alpha*-methyl-glutamic acid, or cysteine (C), or is absent;
X17 is aspartic acid (D), glutamine (Q), glutamic acid (E), lysine (K), arginine (R), serine (S), cysteine (C), or valine (V), or is absent;
X18 is alanine (A), aspartic acid (D), glutamine (Q), glutamic acid (E), arginine (R), valine (V), or cysteine (C), or is absent;
X19 is alanine (A), arginine (R), serine (S), valine (V), or cysteine (C), or is absent;
X20 is lysine (K), histidine (H), glutamic acid (E), glutamine (Q), aspartic acid (D), arginine (R), *alpha*-methyl-glutamic acid, or cysteine (C), or is absent;
X21 is aspartic acid (D), glutamic acid (E), leucine (L), valine (V), or cysteine (C), or is absent;
X23 is isoleucine (I), valine (V), or arginine (R), or is absent;
X24 is valine (V), arginine (R), alanine (A), cysteine (C), glutamic acid (E), lysine (K), glutamine (Q), *alpha*-methyl-glutamic acid, or leucine (L), or is absent;
X27 is isoleucine (I), valine (V), alanine (A), lysine (K), methionine (M), glutamine (Q), or arginine (R), or is absent;
X28 is glutamine (Q), lysine (K), asparagine (N), or arginine (R), or is absent; and
X30 is cysteine (C) or is absent (with the proviso that when the amino acid sequence of General Formula 1 is the same as SEQ ID NO: 1, it is excluded).

The composition according to any one of the above specific embodiments is characterized in that, in General Formula 1, X1 is histidine (H), tryptophan (W), or tyrosine (Y), or is absent;
X2 is serine (S) or Aib (aminoisobutyric acid);
X7 is threonine (T), valine (V) or cysteine (C);
X10 is tyrosine (Y) or cysteine (C);
X12 is lysine (K) or cysteine (C);
X13 is tyrosine (Y) or cysteine (C);
X14 is leucine (L) or cysteine (C);
X15 is aspartic acid (D) or cysteine (C);
X16 is glutamic acid (E), serine (S), or cysteine (C);
X17 is aspartic acid (D), glutamic acid (E), lysine (K), arginine (R), serine (S), cysteine (C), or valine (V);
X18 is aspartic acid (D), glutamic acid (E), arginine (R), or cysteine (C);
X19 is alanine (A), or cysteine (C);
X20 is glutamine (Q), aspartic acid (D), lysine (K), or cysteine (C);
X21 is aspartic acid (D), glutamic acid (E), leucine (L), valine (V), or cysteine (C);
X23 is isoleucine (I), valine (V), or arginine (R);
X24 is valine (V), arginine (R), alanine (A), glutamic acid (E), lysine (K), glutamine (Q), or leucine (L);
X27 is isoleucine (I), valine (V), alanine (A), methionine (M), glutamine (Q) or arginine (R);
X28 is glutamine (Q), lysine (K), asparagine (N), or arginine (R); and
X30 is cysteine (C) or is absent.

The composition according to any one of the above specific embodiments is characterized in that, in General Formula 1,
X1 is histidine (H), tryptophan (W), or tyrosine (Y);
X2 is serine (S) or Aib (aminoisobutyric acid);
X7 is cysteine (C), threonine (T), or valine (V);
X10 is tyrosine (Y) or cysteine (C);
X12 is lysine (K) or cysteine (C);
X13 is tyrosine (Y) or cysteine (C);
X14 is leucine (L) or cysteine (C);
X15 is aspartic acid (D) or cysteine (C);
X16 is glutamic acid (E), serine (S), or cysteine (C);
X17 is glutamic acid (E), lysine (K), arginine (R), cysteine (C), or valine (V);
X18 is arginine (R) or cysteine (C);
X19 is alanine (A) or cysteine (C);
X20 is glutamine (Q) or lysine (K);
X21 is aspartic acid (D), glutamic acid (E), valine (V), or cysteine (C);
X23 is valine (V);
X24 is valine (V) or glutamine (Q);
X27 is methionine (M);
X28 is asparagine (N) or arginine (R); and
X30 is cysteine (C) or is absent.

The composition according to any one of the above specific embodiments is characterized in that, in General Formula 1,
X1 is tyrosine (Y);
X2 is Aib (aminoisobutyric acid);
X7 is cysteine (C), threonine (T), or valine (V);
X10 is tyrosine (Y) or cysteine (C);
X12 is lysine (K);
X13 is tyrosine (Y) or cysteine (C);
X14 is leucine (L) or cysteine (C);
X15 is aspartic acid (D) or cysteine (C);
X16 is glutamic acid (E), serine (S), or cysteine (C);
X17 is lysine (K), arginine (R), cysteine (C), or valine (V);
X18 is arginine (R) or cysteine (C);
X19 is alanine (A) or cysteine (C);
X20 is glutamine (Q) or lysine (K);
X21 is aspartic acid (D), glutamic acid (E), or cysteine (C);
X23 is valine (V);
X24 is glutamine (Q);
X27 is methionine (M);
X28 is asparagine (N) or arginine (R); and
X30 is cysteine (C) or is absent.

The composition according to any one of the above specific embodiments is characterized in that, in General Formula 1,
X1 is histidine (H), tryptophan (W), or tyrosine (Y), or is absent;
X2 is serine (S) or Aib (aminoisobutyric acid);
X7 is threonine (T), valine (V), or cysteine (C);
X10 is tyrosine (Y) or cysteine (C);
X12 is lysine (K) or cysteine (C);
X13 is tyrosine (Y) or cysteine (C);
X14 is leucine (L) or cysteine (C);
X15 is aspartic acid (D) or cysteine (C);
X16 is glutamic acid (E), serine (S), or cysteine (C);
X17 is aspartic acid (D), glutamic acid (E), lysine (K), arginine (R), serine (S), cysteine (C), or valine (V);
X18 is aspartic acid (D), glutamic acid (E), arginine (R), or cysteine (C);
X19 is alanine (A) or cysteine (C);
X20 is glutamine (Q), aspartic acid (D), or lysine (K);
X21 is aspartic acid (D) or glutamic acid (E);
X23 is valine (V);
X24 is valine (V) or glutamine (Q);
X27 is isoleucine (I) or methionine (M);
X28 is asparagine (N) or arginine (R); and
X30 is cysteine (C) or is absent.

The composition according to any one of the above specific embodiments is characterized in that, in General Formula 1,
X1 is tyrosine (Y);
X2 is Aib (aminoisobutyric acid);
X7 is threonine (T);
X10 is tyrosine (Y);
X12 is lysine (K);
X13 is tyrosine (Y);
X14 is leucine (L);
X15 is aspartic acid (D) or cysteine (C);
X16 is glutamic acid (E), serine (S), or cysteine (C);
X17 is lysine (K) or arginine (R);
X18 is arginine (R);
X19 is alanine (A);
X20 is glutamine (Q), cysteine (C), or lysine (K);
X21 is aspartic acid (D), cysteine (C), valine (V), or glutamic acid (E);
X23 is valine (V) or arginine (R);
X24 is glutamine (Q) or leucine (L);
X27 is methionine (M);
X28 is asparagine (N) or arginine (R); and
X30 is absent.

The composition according to any one of the above specific embodiments is characterized in that the glucagon analog includes an amino acid sequence of the following General Formula 2: 
Y–Aib–QGTF–X7–SD–X10–S–X12–Y–L–X15–X16–X17–R–A–X20–X21–
F–V–X24–W–L–M–N–T–X30 (General Formula 2, SEQ ID NO: 47)
 in General Formula 2,
X7 is threonine (T), valine (V), or cysteine (C);
X10 is tyrosine (Y) or cysteine (C);
X12 is lysine (K) or cysteine (C);
X15 is aspartic acid (D) or cysteine (C);
X16 is glutamic acid (E) or serine (S);
X17 is lysine (K) or arginine (R);
X20 is glutamine (Q) or lysine (K);
X21 is aspartic acid (D) or glutamic acid (E);
X24 is valine (V) or glutamine (Q); and
X30 is cysteine (C) or is absent.

The composition according to any one of the above specific embodiments is characterized in that at least one amino acid pair among the amino acid pairs of X10 and X14, X12 and X16, X16 and X20, X17 and X21, X20 and X24, and X24 and X28 in General Formula 1 forms a ring, respectively.

The composition according to any one of the above specific embodiments is characterized in that each amino acid of at least one amino acid pair among the amino acid pairs of X10 and X14, X12 and X16, X16 and X20, X17 and X21, X20 and X24, and X24 and X28 in General Formula 1 is substituted with glutamic acid or lysine, which is capable of forming a ring.

The liquid formulation according to any one of the above specific embodiments is characterized in that, in the long-acting conjugate of the glucagon analog, pl of the glucagon analog is 6 to 7, and *in vitro* activity thereof relative to that of the native glucagon is 200% or more. The composition according to any one of the above specific embodiments is characterized in that the glucagon analog includes an amino acid sequence selected from the group consisting of SEQ ID NOS: 2 to 45.

The composition according to any one of the above specific embodiments is characterized in that the glucagon analog includes an amino acid sequence of SEQ ID NO: 37.

The composition according to any one of the above specific embodiments is characterized in that the insulin is natural insulin; or an insulin analog obtained by alteration selected from the group consisting of substitution, addition, deletion, modification, and a combination thereof in one or more amino acids of the native insulin.

The composition according to any one of the above specific embodiments is characterized in that the insulin analog is an insulin analog obtained by substituting another amino acid for, or by deleting one or more amino acids selected from the group consisting of an amino acid at position 1, an amino acid at position 2, an amino acid at position 3, an amino acid at position 5, an amino acid at position 8, an amino acid at position 10, an amino acid at position 12, an amino acid at position 16, an amino acid at position 23, an amino acid at position 24, an amino acid at position 25, an amino acid at position 26, an amino acid at position 27, an amino acid at position 28, an amino acid at position 29, and an amino acid at position 30 of a B-chain of the natural insulin, and an amino acid at position 1, an amino acid at position 2, an amino acid at position 5, an amino acid at position 8, an amino acid at position 10, an amino acid at position 12, an amino acid at position 14, an amino acid at position 16, an amino acid at position 17, an amino acid at position 18, an amino acid at position 19, and an amino acid at position 21 of an A-chain of the natural insulin.

The composition according to any one of the above specific embodiments is characterized in that the insulin analog includes an A-chain of SEQ ID NO: 48 represented by the following General Formula 3 and a B-chain of SEQ ID NO: 49 represented by the following General Formula 4: 
[General Formula 3]

Xaa1–Xaa2–Val–Glu–Xaa5–Cys–Cys–Thr–Ser–Ile–Cys–Xaa12–Leu–

Xaa14–Gln–Xaa16–Glu–Asn–Xaa19–Cys–Xaa21 (SEQ ID NO: 48)
 in General Formula 3,
Xaa1 is alanine, glycine, glutamine, histidine, glutamic acid, or asparagine;
Xaa2 is alanine or isoleucine;
Xaa5 is alanine, glutamic acid, glutamine, histidine, or asparagine;
Xaa12 is alanine, serine, glutamine, glutamic acid, histidine, or asparagine;
Xaa14 is alanine, tyrosine, glutamic acid, histidine, lysine, aspartic acid, or asparagine;
Xaa16 is alanine, leucine, tyrosine, histidine, glutamic acid, or asparagine;
Xaa19 is alanine, tyrosine, serine, glutamic acid, histidine, threonine, or asparagine; and
Xaa21 is asparagine, glycine, histidine, or alanine; 
[General Formula 4]

Phe–Val–Asn–Gln–His–Leu–Cys–Xaa8–Ser–His–Leu–Val–Glu–Ala–Leu–

Xaa16–Leu–Val–Cys–Gly–Glu–Arg–Xaa23–Xaa24–Xaa25–Tyr–Xaa27–Xaa28–

Lys–Thr (SEQ ID NO: 49)
in General Formula 4,
Xaa8 is alanine or glycine;
Xaa16 is tyrosine, glutamic acid, serine, threonine, or aspartic acid, or is absent;
Xaa23 is glycine or alanine;
Xaa24 is alanine or phenylalanine;
Xaa25 is alanine, phenylalanine, aspartic acid, or glutamic acid, or is absent;
Xaa27 is threonine or is absent; and
Xaa28 is proline, glutamic acid, or aspartic acid, or is absent
(here, with the proviso that when a peptide includes an A-chain of SEQ ID NO: 48 and a B-chain of SEQ ID NO: 49, it is excluded).

The composition according to any one of the above specific embodiments is characterized in that the insulin analog is prepared by substituting alanine for one amino acid selected from the group consisting of an amino acid at position 8, an amino acid at position 23, an amino acid at position 24, and an amino acid at position 25 of a B-chain of the natural insulin, and an amino acid at position 1, an amino acid at position 2, and an amino acid at position 19 of the A-chain of the natural insulin, or by substituting glutamic acid or asparagine for an amino acid at position 14 of the A-chain of the natural insulin.

The composition according to any one of the above specific embodiments is characterized in that the insulin analog includes an amino acid sequence selected from the group consisting of SEQ ID NOS: 51, 53, 55, 57, 59, 61, 63, 65, and 67.

The composition according to any one of the above specific embodiments is characterized in that the insulin analog is obtained by substituting glutamic acid for an amino acid at position 16 or by deleting an amino acid at position 25 of the B-chain of the natural insulin; or by substituting glutamic acid or alanine for an amino acid at position 14 of the A-chain of the natural insulin.

The composition according to any one of the above specific embodiments is characterized in that the insulin analog includes an amino acid sequence of SEQ ID NO: 69 or 71.

The composition according to any one of the above specific embodiments is characterized in that the insulin analog is obtained by substituting glutamic acid, serine, threonine, or aspartic acid for an amino acid at position 16 of the B-chain of the natural insulin; by substituting aspartic acid or glutamic acid for an amino acid at position 25 of the B-chain of the natural insulin; or by substituting histidine, lysine, alanine, or aspartic acid for an amino acid at position 14 of the A-chain of the natural insulin; or by substituting glutamic acid, serine, or threonine for an amino acid at position 19 of the A-chain of the natural insulin.

The composition according to any one of the above specific embodiments is characterized in that the insulin analog includes an amino acid sequence selected from the group consisting of SEQ ID NOS: 99, 101, 103, 105, 107, 109, 111, 113, 115, 117, 119, 121, and 123.

The composition according to any one of the above specific embodiments is characterized in that the insulin analog is in the form of two polypeptide chains consisting of an A-chain of SEQ ID NO: 48 represented by General Formula 3 and a B-chain of SEQ ID NO: 49 represented by General Formula 4.

The composition according to any one of the above specific embodiments is characterized in that the A-chain and B-chain are linked to each other via a disulfide bond.

The composition according to any one of the above specific embodiments is characterized in that the conjugate is represented by the following Chemical Formula 1:

### [Chemical Formula 1]

X-La-F

wherein X is the insulin or the glucagon;
L is a linker;
a is 0 or a natural number, provided that when a is 2 or more, each L is independent;
F is a material capable of increasing *in vivo* half-life of X; and
"-" represents a covalent or non-covalent bond.

The composition according to any one of the above specific embodiments is characterized in that F is selected from the group consisting of a high-molecular-weight polymer, a fatty acid, cholesterol, albumin and a fragment thereof, an albumin-binding material, a polymer of repeating units of a particular amino acid sequence, an antibody, an antibody fragment, an FcRn-binding material, an *in vivo* connective tissue, a nucleotide, fibronectin, transferrin, a saccharide, heparin, and elastin.

The composition according to any one of the above specific embodiments is characterized in that the high-molecular-weight polymer is selected from the group consisting of polyethylene glycol, polypropylene glycol, an ethylene glycol-propylene glycol copolymer, polyoxyethylated polyol, polyvinyl alcohol, a polysaccharide, polyvinyl ethyl ether, a biodegradable polymer, a lipid polymer, chitin, hyaluronic acid, an oligonucleotide, and a combination thereof.

The composition according to any one of the above specific embodiments is characterized in that F is an immunoglobulin Fc region.

The composition according to any one of the above specific embodiments is characterized in that F is an IgG Fc region.

The composition according to any one of the above specific embodiments is characterized in that the immunoglobulin Fc region is aglycosylated.

The composition according to any one of the above specific embodiments is characterized in that the immunoglobulin Fc region is selected from the group consisting of (a) a CH1 domain, a CH2 domain, a CH3 domain, and a CH4 domain; (b) a CH1 domain and a CH2 domain; (c) a CH1 domain and a CH3 domain; (d) a CH2 domain and a CH3 domain; (e) a combination between one or two or more domains among a CH1 domain, a CH2 domain, a CH3 domain, and a CH4 domain and an immunoglobulin hinge region or a part of the hinge region; and (f) a dimer between each domain of the heavy chain constant region and the light chain constant region.

The composition according to any one of the above specific embodiments is characterized in that the immunoglobulin Fc region is an immunoglobulin Fc region, in which the region capable of forming a disulfide bond is deleted, in which a part of the amino acid(s) in the N-terminus is deleted, in which a methionine residue is added to the N-terminus of native Fc, in which a complement-binding site is deleted, or in which an antibody-dependent cell-mediated cytotoxicity (ADCC) site is deleted.

The composition according to any one of the above specific embodiments is characterized in that the immunoglobulin Fc region is an immunoglobulin Fc region derived from IgG, IgA, IgD, IgE, or IgM.

The composition according to any one of the above specific embodiments is characterized in that the immunoglobulin Fc region is a hybrid of a domain having a different origin derived from an immunoglobulin selected from the group consisting of IgG, IgA, IgD, IgE, and IgM.

The composition according to any one of the above specific embodiments is characterized in that L is selected from the group consisting of a peptide, a fatty acid, saccharide, a high-molecular-weight polymer, a low-molecular-weight compound, a nucleotide, and a combination thereof.

The composition according to any one of the above specific embodiments is characterized in that the high-molecular-weight polymer is selected from the group consisting of polyethylene glycol, polypropylene glycol, an ethylene glycol-propylene glycol copolymer, polyoxyethylated polyol, polyvinyl alcohol, a polysaccharide, polyvinyl ethyl ether, a biodegradable polymer, a lipid polymer, chitin, hyaluronic acid, an oligonucleotide, and a combination thereof.

The composition according to any one of the above specific embodiments is characterized in that L is polyethylene glycol.

To achieve the present invention, another aspect provides a pharmaceutical composition for preventing or treating hypoglycemia, the pharmaceutical composition including insulin and glucagon.

The composition according to one specific embodiment is characterized in that the insulin is linked to a biocompatible material capable of increasing *in vivo* half-life thereof to be in the form of a long-acting conjugate.

The composition according to the above specific embodiment is characterized in that the glucagon is linked to a biocompatible material capable of increasing *in vivo* half-life thereof to be in the form of a long-acting conjugate.

The composition according to any one of the above specific embodiments is characterized in that the insulin is linked to a biocompatible material capable of increasing *in vivo* half-life thereof to be in the form of a long-acting conjugate, and the glucagon is linked to a biocompatible material capable of increasing *in vivo* half-life thereof to be in the form of a long-acting conjugate.

To achieve the present invention, still another aspect provides a composition for improving side effects of insulin, the composition including insulin and glucagon.

The composition according to one specific embodiment is characterized in that the insulin is linked to a biocompatible material capable of increasing *in vivo* half-life thereof to be in the form of a long-acting conjugate.

The composition according to the above specific embodiment is characterized in that the glucagon is linked to a biocompatible material capable of increasing *in vivo* half-life thereof to be in the form of a long-acting conjugate.

The composition according to any one of the above specific embodiments is characterized in that the insulin is linked to a biocompatible material capable of increasing *in vivo* half-life thereof to be in the form of a long-acting conjugate, and the glucagon is linked to a biocompatible material capable of increasing *in vivo* half-life thereof to be in the form of a long-acting conjugate.

To achieve the present invention, still another aspect provides a complex formulation for improving hypoglycemia of a patient with an insulin-related disease, the complex formulation including insulin and glucagon.

The complex formulation according to one specific embodiment is characterized in that the insulin is linked to a biocompatible material capable of increasing *in vivo* half-life thereof to be in the form of a long-acting conjugate.

The complex formulation according to the above specific embodiment is characterized in that the glucagon is linked to a biocompatible material capable of increasing *in vivo* half-life thereof to be in the form of a long-acting conjugate.

The complex formulation according to any one of the above specific embodiments is characterized in that the insulin is linked to a biocompatible material capable of increasing *in vivo* half-life thereof to be in the form of a long-acting conjugate, and the glucagon is linked to a biocompatible material capable of increasing *in vivo* half-life thereof to be in the form of a long-acting conjugate.

The complex formulation according to any one of the above specific embodiments is characterized in that the hypoglycemia is a side effect of insulin.

The complex formulation according to any one of the above specific embodiments is characterized in that it suppresses weight gain.

The complex formulation according to any one of the above specific embodiments is characterized in that it includes the insulin or the long-acting conjugate thereof and the glucagon or the long-acting conjugate thereof at a weight ratio of 0.1:1 to 100:1.

### [Advantageous Effects]

A composition or a complex formulation, each including insulin or a long-acting conjugate thereof and glucagon or a long-acting conjugate thereof of the present invention provides a novel combination therapy capable of alleviating side effects caused by insulin administration, such as weight gain or hypoglycemia, while exhibiting a prophylactic or therapeutic effect on an insulin-related disease, e.g., diabetes.

### [Description of Drawings]

FIG. 1 shows changes in blood glucose levels according to combined administration of a long-acting insulin conjugate and a long-acting glucagon conjugate;
FIG. 2 shows changes (AUC) in blood glucose levels according to combined administration of a long-acting insulin conjugate and a long-acting glucagon conjugate; and
FIG. 3 shows changes in the body weight according to combined administration of a long-acting insulin conjugate and a long-acting glucagon conjugate.

### [Best Mode]

Hereinafter, the present invention will be described in more detail. Meanwhile, each of the explanations and embodiments disclosed in the present invention may be applied to other explanations and embodiments. That is, all combinations of various elements disclosed in the present invention belong to the scope of the present invention. Additionally, the scope of the present invention should not be limited by the specific descriptions described hereinbelow.

Throughout the disclosure of the present invention, not only the conventional 1-letter codes and 3-letter codes for amino acids present in nature but also the 3-letter codes generally used for other amino acids, such as Aib (α-aminoisobutyric acid), Sar (*N*-methylglycine), and alpha-methyl-glutamic acid (α-methyl-glutamic acid), are used. Further, the amino acids mentioned in abbreviation in the present disclosure are described according to the IUPAC-IUB Nomenclature.

| | | | |
|---|---|---|---|
| alanine | Ala, A | arginine | Arg, R |
| asparagine | Asn, N | aspartic acid | Asp, D |
| cysteine | Cys, C | glutamic acid | Glu, E |
| glutamine | Gln, Q | glycine | Gly, G |
| histidine | His, H | isoleucine | Ile, I |
| leucine | Leu, L | lysine | Lys, K |
| methionine | Met, M | phenylalanine | Phe, F |
| proline | Pro, P | serine | Ser, S |
| threonine | Thr, T | tryptophan | Trp, W |
| tyrosine | Tyr, Y | valine | Val, V |

To achieve the present invention, an aspect provides a pharmaceutical composition for preventing or treating an insulin-related disease, the pharmaceutical composition including insulin and glucagon.

Specifically, the composition may include
(i) insulin and glucagon;
(ii) a long-acting insulin conjugate, in which insulin is linked to a biocompatible material capable of increasing *in vivo* half-life thereof, and glucagon;
(iii) insulin; and a long-acting glucagon conjugate, in which glucagon is linked to a biocompatible material capable of increasing *in vivo* half-life thereof; or
(iv) the long-acting insulin conjugate and the long-acting glucagon conjugate.

The pharmaceutical composition for preventing or treating an insulin-related disease of the present invention includes insulin or the long-acting conjugate thereof and glucagon or the long-acting conjugate thereof, and may alleviate side effects caused by insulin administration (*e.g.*, weight gain or hypoglycemia), while exhibiting a prophylactic or therapeutic effect on an insulin-related disease caused by deficiency, lack, or malfunction of insulin.

In one exemplary embodiment of the present invention, blood glucose-lowering, weight gain-suppressing, and hypoglycemia-alleviating effects were confirmed in db/db mice administered with the composition including the insulin or the long-acting conjugate thereof and the glucagon or the long-acting conjugate thereof, and thus it was demonstrated that the pharmaceutical composition according to the present invention may maintain insulin efficacy while improving side effects thereof.

In one specific embodiment of the present invention, the composition may provide therapeutic use of a combination of the insulin or the long-acting conjugate thereof and the glucagon or the long-acting conjugate thereof.

As used herein, the "composition" may be used interchangeably with a "combination" including the insulin or the long-acting conjugate thereof and the glucagon or the long-acting conjugate thereof. The composition may be provided in the form of a kit.

As used herein, the "combination" has use of combined administration of the insulin or the long-acting conjugate thereof and the glucagon or the long-acting conjugate thereof, and it may be understood that the combination has the same meaning as "combined use". The combination may be administered a) in a mixture form, in which (i) the insulin or the long-acting conjugate thereof and (ii) the glucagon or the long-acting conjugate thereof are mixed; or b) in a separate form, in which (i) the insulin or the long-acting conjugate thereof and (ii) the glucagon or the long-acting conjugate thereof are separately administered, but is not limited thereto.

When the insulin or the long-acting conjugate thereof and the glucagon or the long-acting conjugate thereof are in a separate form, the insulin or the long-acting conjugate thereof and the glucagon or the long-acting conjugate thereof may be formulated into separate preparations to be administered simultaneously, individually, sequentially, or in reverse order.

In the present invention, the combined administration not only means simultaneous administration, but should also be understood as a dosage form capable of performing a function at a level equal to or higher than the natural function of each substance when the insulin or the long-acting conjugate thereof and the glucagon or the long-acting conjugate thereof act together on an individual. Therefore, in the present application, when the term "combined" is used, it should be understood that they are administered simultaneously, individually, sequentially, or in reverse order. When the administration is performed sequentially, in reverse order, or individually, the order of administration is not particularly limited. However, the interval to the second administration should be such that the beneficial effects of the combined administration are not lost.

As used herein, the term "composition" may be the combination itself including the insulin or the long-acting conjugate thereof and the glucagon or the long-acting conjugate thereof, or a composition including the combination and the therapeutic use, but is not limited thereto. For example, the composition may be a composition having prophylactic or therapeutic use for insulin-related diseases, but is not limited thereto.

The composition according to the present invention may be for combined administration of the insulin or the long-acting conjugate thereof and the glucagon or the long-acting conjugate thereof, and the insulin or the long-acting conjugate thereof and the glucagon or the long-acting conjugate thereof may be formulated into a single preparation or individual preparations. Specifically, the insulin or the long-acting conjugate thereof and the glucagon or the long-acting conjugate thereof may be administered simultaneously, individually, sequentially, or in reverse order, but is not limited thereto.

As used herein, the term "kit" may include the combination or composition according to the present invention for combined administration of the insulin or the long-acting conjugate thereof and the glucagon or the long-acting conjugate thereof. Specifically, the kit according to the present invention may include the insulin or the conjugate thereof and the glucagon or long-acting conjugate thereof, which are formulated into a single preparation, or the insulin or the conjugate thereof and the glucagon or the conjugate thereof, which are formulated into individual preparations, and may further include a material needed for the combined administration of the two materials, but is not limited thereto.

The insulin and the glucagon include various materials having a significant level of activity for insulin and glucagon, *e.g.,* a material in the form of a compound or a peptide.

As used herein, the term "insulin-related disease" refers to a disease caused by abnormalities in the blood glucose control function of insulin, such as deficiency, lack, or malfunction of insulin, and any disease is included in the scope of the present invention, as long as the disease is expected to receive a prophylactic or therapeutic effect due to the administration of insulin. Specifically, the insulin-related disease may be selected from the group consisting of insulin-resistant diseases, diabetes, hyperglycemia, and obesity, but is not limited thereto.

When insulin is administered for the treatment of the insulin-related disease, unintended side effects appear, such as hypoglycemia or weight gain, even though the desired therapeutic effect may be obtained, and thus there is a problem in that another disease and pain are caused in patients. The pharmaceutical composition according to the present invention, in which insulin and glucagon are used in combination, may alleviate hypoglycemia, which is a side effect of insulin, and may suppress weight gain, while exhibiting a therapeutic effect on insulin-related diseases.

The pharmaceutical composition of the present invention may include the insulin or the long-acting conjugate thereof and the glucagon or the long-acting conjugate thereof in a content ratio capable of alleviating side effects while exhibiting a therapeutic effect on insulin-related diseases. Specifically, they may be included at a weight ratio of 0.1:1 to 100:1, or at a molar ratio of 1:1 to 30:1, 1:1 to 20:1, 1:1 to 16:1, but are not limited thereto.

The pharmaceutical composition according to the present invention may include a specific ratio of the insulin or the long-acting conjugate thereof and the glucagon or the long-acting conjugate thereof, thereby exhibiting an excellent blood glucose control effect while suppressing side effects. When the weight ratio is outside the above range, for example, when the insulin or the long-acting conjugate thereof is included in an excess amount, there is a high risk of significantly developing side effects of insulin, and when the glucagon or the long-acting conjugate thereof is included in an excess amount, there is a problem in that the therapeutic effect on insulin-related diseases may decrease.

As used herein, the term "insulin" refers to a kind of hormone secreted from beta cells of the pancreas, and generally has a role in regulating blood glucose in the body by promoting intracellular glucose uptake and inhibiting breakdown of fat. Proinsulin in the form of a precursor having no blood glucose control function is processed to insulin having a blood glucose control function. Insulin consists of two polypeptide chains, *i.e.*, an A-chain and a B-chain, including 21 and 30 amino acid residues, respectively, which are connected to each other via two disulfide bridges. The A-chain and B-chain of natural insulin include amino acid sequences represented by the following SEQ ID NOS: 124 and 125, respectively.

A-chain:

Gly–Ile–Val–Glu–Gln–Cys–Cys–Thr–Ser–Ile–Cys–Ser–Leu–Tyr–Gln–Leu–

Glu–Asn–Tyr–Cys–Asn (SEQ ID NO: 124)

B-chain:

Phe–Val–Asn–Gln–His–Leu–Cys–Gly–Ser–His–Leu–Val–Glu–Ala–Leu–
Tyr–Leu–Val–Cys–Gly–Glu–Arg–Gly–Phe–Phe–Tyr–Thr–Pro–Lys–Thr (SEQ ID
NO: 125)

As used herein, the term "proinsulin" refers to a precursor molecule of insulin. The proinsulin may include insulin an A-chain and a B-chain, and a C-peptide therebetween. The proinsulin may be human proinsulin.

In the present invention, the insulin may be natural insulin, or may be an analog, a derivative, or a fragment, which is obtained by alteration selected from the group consisting of substitution, addition, deletion, modification, and a combination thereof in one or more amino acids of the natural insulin, but is not limited thereto.

As used herein, the term "insulin analog" refers to a non-natural insulin different from the natural insulin.

The insulin analog includes an analog obtained by modifying part of amino acids of the natural insulin by addition, deletion, or substitution. For example, the insulin analog may be an insulin analog obtained by substituting another amino acid for, or deleting one or more amino acids selected from the group consisting of an amino acid at position 1, an amino acid at position 2, an amino acid at position 3, an amino acid at position 5, an amino acid at position 8, an amino acid at position 10, an amino acid at position 12, an amino acid at position 16, an amino acid at position 23, an amino acid at position 24, an amino acid at position 25, an amino acid at position 26, an amino acid at position 27, an amino acid at position 28, an amino acid at position 29, and an amino acid at position 30 of the B-chain of the natural insulin, and an amino acid at position 1, an amino acid at position 2, an amino acid at position 5, an amino acid at position 8, an amino acid at position 10, an amino acid at position 12, an amino acid at position 14, an amino acid at position 16, an amino acid at position 17, an amino acid at position 18, an amino acid at position 19, and an amino acid at position 21 of the A-chain of the natural chain, but is not limited thereto. The amino acid substitution may be generally made on the basis of similarity in polarity, charge, solubility, hydrophobicity, hydrophilicity, and/or the amphipathic nature of the residues. Conservative substitutions with amino acids having similar properties may be expected to exhibit the same or similar activity.

The insulin analog of the present invention may refer to the contents of Korean Patent Publication No. 10-2014-0106452 or 10-2017-0026284.

The insulin analog applied in the present invention may be in the form of a single chain or two polypeptide chains, and more preferably in the form of two polypeptide chains, but is not particularly limited thereto. The two polypeptide chains may consist of two polypeptides, a polypeptide corresponding to the A-chain of natural insulin and a polypeptide corresponding to the B-chain of natural insulin. Here, the peptide corresponding to the A-chain or B-chain of natural insulin may include at least 60% or more, 65% or more, 70% or more, 75% or more, 80% or more, 85% or more, 90% or more, 91% or more, 92% or more, 93% or more, 94% or more, or 95% or more sequence identity when comparing the sequence identity between any one chain of the two polypeptide chains with the A-chain or B-chain of natural insulin, but is not particularly limited thereto, and those skilled in the art may easily identify by comparing the sequence constituting the two polypeptide chains with the sequence of the A-chain or B-chain of natural insulin.

As used herein, the term "homology" is intended to indicate the degree of similarity to an amino acid sequence of a wild-type protein or a nucleotide sequence encoding the same, and includes sequences having a homology of the above percentage or higher with the amino acid sequence or nucleotide sequence of the present invention. The homology may be determined by comparing the two given sequences with the naked eye, but may be determined using a bioinformatics algorithm which enables the analysis of a homology by arranging the subject sequences for comparison. The homology between the two given amino acid sequences may be indicated as a percentage. The useful automated algorithm is available for use in GAP, BESTFIT, FASTA, and TFASTA computer software modules of the Wisconsin Genetics Software Package (Genetics Computer Group, Madison, WI, USA). The arrangement algorithm automated in the above modules includes sequence arrangement algorithms by Needleman & Wunsch, Pearson & Lipman, and Smith & Waterman. Other useful algorithms for sequence arrangement and homology determination are automated in software including FASTP, BLAST, BLAST2, PSIBLAST, and CLUSTAL W.

The information of the sequences of insulin and glucagon used in the present invention and the nucleotide sequences encoding the same may be obtained from a public database, such as the NCBI, *etc.*

In one specific exemplary embodiment, the insulin analog of the present invention may be a composition including an A-chain of SEQ ID NO: 48 represented by the following General Formula 3 and a B-chain of SEQ ID NO: 49 represented by the following General Formula 4, but is not limited thereto: 
[General Formula 3]

Xaa1–Xaa2–Val–Glu–Xaa5–Cys–Cys–Thr–Ser–Ile–Cys–Xaa12–Leu–

Xaa14–Gln–Xaa16–Glu–Asn–Xaa19–Cys–Xaa21 (SEQ ID NO: 48)
 in General Formula 3,
Xaa1 is alanine, glycine, glutamine, histidine, glutamic acid, or asparagine;
Xaa2 is alanine or isoleucine;
Xaa5 is alanine, glutamic acid, glutamine, histidine, or asparagine;
Xaa12 is alanine, serine, glutamine, glutamic acid, histidine, or asparagine;
Xaa14 is alanine, tyrosine, glutamic acid, histidine, lysine, aspartic acid, or asparagine;
Xaa16 is alanine, leucine, tyrosine, histidine, glutamic acid, or asparagine;
Xaa19 is alanine, tyrosine, serine, glutamic acid, histidine, threonine, or asparagine; and
Xaa21 is asparagine, glycine, histidine, or alanine; 
[General Formula 4]
Phe–Val–Asn–Gln–His–Leu–Cys–Xaa8–Ser–His–Leu–Val–Glu–Ala–Leu–
Xaa16–Leu–Val–Cys–Gly–Glu–Arg–Xaa23–Xaa24–Xaa25–Tyr–Xaa27–Xaa28–
Lys–Thr (SEQ ID NO: 49)
and in General Formula 4,
Xaa8 is alanine or glycine;
Xaa16 is tyrosine, glutamic acid, serine, threonine, or aspartic acid, or is absent;
Xaa23 is glycine or alanine;
Xaa24 is alanine or phenylalanine;
Xaa25 is alanine, phenylalanine, aspartic acid, or glutamic acid, or is absent;
Xaa27 is threonine or is absent; and
Xaa28 is proline, glutamic acid, or aspartic acid, or is absent
(wherein a peptide including an A-chain of SEQ ID NO: 48 and a B-chain of SEQ ID NO: 49 is excluded).

More specifically, the insulin analog may be an insulin analog obtained by substituting alanine for one or more amino acids selected from the group consisting of an amino acid at position 8, an amino acid at position 23, an amino acid at position 24, and an amino acid at position 25 of the B-chain of the natural insulin, and an amino acid at position 1, an amino acid at position 2, and an amino acid at position 19 of the A-chain of the natural insulin, or by substituting glutamic acid or asparagine for an amino acid at position 14 of the A-chain of the natural insulin, and in particular, the insulin analog may include an amino acid sequence selected from the group consisting of SEQ ID NOS: 51, 53, 55, 57, 59, 61, 63, 65, and 67, or may consist of the amino acid sequence, but is not limited thereto.

Further, the insulin analog may be an insulin analog obtained by substituting glutamic acid for an amino acid at position 16 of the B-chain of the natural insulin; by deleting an amino acid at position 25 of the B-chain of the natural insulin; or by substituting glutamic acid or alanine for an amino acid at position 14 of the A-chain of the natural insulin, and in particular, the insulin analog may include an amino acid sequence of SEQ ID NO: 69 or 71, or may consist of the amino acid sequence, but is not limited thereto.

Further, the insulin analog may be an insulin analog obtained by substituting glutamic acid, serine, threonine, or aspartic acid for an amino acid at position 16 of the B-chain of the natural insulin; by substituting aspartic acid or glutamic acid for an amino acid at position 25 of the B-chain of the natural insulin; by substituting histidine, lysine, alanine, or aspartic acid for an amino acid at position 14 of the A-chain of the natural insulin; or by substituting glutamic acid, serine, or threonine for an amino acid at position 19 of the A-chain of the natural insulin, and in particular, the insulin analog may include an amino acid sequence selected from the group consisting of SEQ ID NOS: 99, 101, 103, 105, 107, 109, 111, 113, 115, 117, 119, 121, and 123, or may consist of the amino acid sequence, but is not limited thereto.

Although described as "consisting of a specific SEQ ID NO" in the present disclosure, as long as the protein may have an activity identical or corresponding to that of the polypeptide consisting of the amino acid sequence of the corresponding SEQ ID NO, addition of a meaningless sequence upstream or downstream of the amino acid sequence of the corresponding SEQ ID NO, a naturally occurring mutation, or a silent mutation therein is not excluded, and it will be apparent that those having such a sequence addition or mutation are also included in the scope of the present invention.

The above description may be applied to other specific embodiments or other aspects of the present invention, but is not limited thereto.

Meanwhile, the insulin analog of the present invention may be in the form of a single-chain insulin, in which a C-peptide is not removed, and may be a material including an A-chain of SEQ ID NO: 48 represented by General Formula 3 and a B-chain of SEQ ID NO: 49 represented by General Formula 4 and having insulin activity and function.

Further, the insulin analog of the present invention may be obtained by removing the C-peptide from proinsulin including the C-peptide between the A-chain and B-chain. The removal of the C-peptide may be performed by a method known in the art, and specifically, it may be prepared by treatment with trypsin and carboxypeptidase B, but is not limited thereto.

Specifically, the insulin analog of the present invention may consist of an A-chain of SEQ ID NO: 48 represented by General Formula 3 and a B-chain of SEQ ID NO: 49 represented by General Formula 4, and more specifically, the insulin analog may be in the form of two polypeptide chains, in which the A-chain and B-chain are linked to each other via two disulfide bridges, but is not limited thereto.

It is apparent that an insulin analog in the form of two polypeptide chains, which is prepared by removing the C-peptide from the insulin analog in the form of proinsulin, represented by a particular SEQ ID NO in the present invention, is also included in the scope of the present invention.

The insulin analog may include a peptide having one or more different sequences in the amino acid sequence, as compared with natural insulin, a peptide altered by modification of the sequence of natural insulin, and a natural insulin mimetic regulating the blood glucose control function in the living body, like natural insulin. In the present invention, the insulin analog may be used interchangeably with an insulin derivative. Such a natural insulin derivative may have the blood glucose control function in the living body.

Specifically, the insulin derivative may be altered by way of any one method of substitution, addition, deletion, and modification of part of amino acids in the natural insulin, or by way of a combination of the methods.

Specifically, the insulin derivative may have an amino acid sequence having at least 80% or more homology to that of the A-chain or B-chain of natural insulin, and/or may be in the form in which a partial group of one amino acid residue of insulin is chemically substituted (*e.g.*, *alpha*-methylation, *alpha-*hydroxylation), removed (*e.g.,* deamination), or modified (*e.g.*, *N*-methylation), but is not limited thereto.

The derivative of natural insulin applied in the present invention may be prepared by a combination of various methods of preparing derivatives.

Further, such alteration for the preparation of insulin derivatives may include all of the alterations using L-type or D-type amino acids, and/or non-native type amino acids; and/or a modification of a native sequence or post-translational modification (*e.g.*, methylation, acylation, ubiquitination, intramolecular covalent bonding, *etc.*)*.*

Further, the alteration may also include all of those in which one or more amino acids are added to the amino and/or carboxy terminal of insulin.

During the substitution or addition of amino acids, not only the 20 amino acids commonly found in human proteins, but also atypical amino acids or those which do not occur naturally may be used. Commercial sources of atypical amino acids may include Sigma-Aldrich, ChemPep Inc., and Genzyme Pharmaceuticals. The peptides including these amino acids and atypical peptide sequences may be synthesized and purchased from commercial suppliers, *e.g.*, American Peptide Company, Bachem (USA), or Anygen (Korea), but are not particularly limited thereto.

As used herein, the term "fragment of natural insulin, insulin analog, or insulin derivative" refers to a form in which one or more amino acids are removed from the amino and/or carboxy terminal of natural insulin, insulin analog, or natural insulin derivative. Such a fragment may have a blood glucose control function in the body.

Further, the insulin analog of the present invention may be prepared by using preparation methods used in preparing the derivatives and fragments of natural insulin, independently or in a combination thereof.

Specifically, the insulin analog according to the present invention may include alteration of a particular amino acid residue in the A-chain and B-chain of natural insulin, and specifically may include alteration of a particular amino acid residue of the A-chain of natural insulin and/or alteration of a particular amino acid residue of the B-chain of natural insulin.

The pharmaceutical composition according to the present invention may include (i) natural insulin, (ii) an insulin analog, (iii) an insulin derivative, (iv) a fragment thereof, or (v) a combination thereof as the insulin which is one of the active ingredients, as long as it has the blood glucose control function of insulin.

As used herein, the term "glucagon" refers to a kind of hormone secreted from alpha cells of the pancreas, and has a role in promoting breakdown of glycogen to increase glucose levels and to increase blood glucose in the body. The native glucagon may have the following amino acid sequence:

His–Ser–Gln–Gly–Thr–Phe–Thr–Ser–Asp–Tyr–Ser–Lys–Tyr–Leu–Asp–
Ser–Arg–Arg–Ala–Gln–Asp–Phe–Val–Gln–Trp–Leu–Met–Asn–Thr (SEQ ID
NO: 1)

In the present invention, glucagon may be native glucagon, or may be an analog, a derivative, or a fragment obtained by alteration selected from the group consisting of substitution, addition, deletion, modification, and a combination thereof in one or more amino acids of the natural glucagon, but is not limited thereto.

As used herein, the term "glucagon analog" refers to a non-natural glucagon different from the natural glucagon.

The glucagon analog includes an analog obtained by modifying part of amino acids of the natural glucagon by addition, deletion, or substitution.

The glucagon analog of the present invention may include at least 60% or more, 65% or more, 70% or more, 75% or more, 80% or more, 85% or more, 90% or more, 91% or more, 92% or more, 93% or more, 94% or more, or 95% or more sequence identity to that of the native glucagon, and is not limited to a particular sequence, as long as it has the effect of increasing blood glucose. The glucagon analog of the present invention may refer to Korean Patent Publication Nos. 10-2017-003466, 10-2018-0002544, and 10-2016-0082482.

In one specific aspect, the glucagon analog may be a peptide including an amino acid sequence of the following General Formula 1: 
X1–X2–QGTF–X7–SD–X10–S–X12–X13–X14–X15–X16–X17–X18–X19–
X20–X21–F–X23–X24–W–L–X27–X28–T–X30 (General Formula 1, SEQ ID
NO: 46)
 in General Formula 1,
X1 is histidine, desamino-histidyl, dimethyl-histidyl (*N*-dimethyl-histidyl), *beta*-hydroxy imidazopropionyl, 4-imidazoacetyl, *beta*-carboxy imidazopropionyl, tryptophan, or tyrosine, or is absent;
X2 is *alpha*-methyl-glutamic acid (a-methyl-glutamic acid), Aib (aminoisobutyric acid), D-alanine, glycine, Sar (*N*-methylglycine), serine, or D-serine;
X7 is threonine, valine, or cysteine;
X10 is tyrosine or cysteine;
X12 is lysine or cysteine;
X13 is tyrosine or cysteine;
X14 is leucine or cysteine;
X15 is aspartic acid, glutamic acid, or cysteine;
X16 is glutamic acid, aspartic acid, serine, *alpha*-methyl-glutamic acid, or cysteine, or is absent;
X17 is aspartic acid, glutamine, glutamic acid, lysine, arginine, serine, cysteine, or valine, or is absent;
X18 is alanine, aspartic acid, glutamine, glutamic acid, arginine, valine, or cysteine, or is absent;
X19 is alanine, arginine, serine, valine, or cysteine, or is absent;
X20 is lysine, histidine, glutamic acid, glutamine, aspartic acid, arginine, *alpha*-methyl-glutamic acid, or cysteine, or is absent;
X21 is aspartic acid, glutamic acid, leucine, valine, or cysteine, or is absent;
X23 is isoleucine, valine, or arginine, or is absent;
X24 is valine, arginine, alanine, cysteine, glutamic acid, lysine, glutamine, *alpha*-methyl-glutamic acid, or leucine, or is absent;
X27 is isoleucine, valine, alanine, lysine, methionine, glutamine, or arginine, or is absent;
X28 is glutamine, lysine, asparagine, or arginine, or is absent; and X30 is cysteine or is absent
(with the proviso that when the amino acid sequence of General Formula 1 is the same as SEQ ID NO: 1, it is excluded).

Much more specifically, in General Formula 1,
X1 is histidine, tryptophan, or tyrosine, or is absent;
X2 is serine or Aib (aminoisobutyric acid);
X7 is threonine, valine, or cysteine;
X10 is tyrosine or cysteine;
X12 is lysine or cysteine;
X13 is tyrosine or cysteine;
X14 is leucine or cysteine;
X15 is aspartic acid or cysteine;
X16 is glutamic acid, serine, or cysteine;
X17 is aspartic acid, glutamic acid, lysine, arginine, serine, cysteine, or valine;
X18 is aspartic acid, glutamic acid, arginine, or cysteine;
X19 is alanine or cysteine;
X20 is glutamine, aspartic acid, lysine, or cysteine;
X21 is aspartic acid, glutamic acid, leucine, valine, or cysteine;
X23 is isoleucine, valine, or arginine;
X24 is valine, arginine, alanine, glutamic acid, lysine, glutamine, or leucine;
X27 is isoleucine, valine, alanine, methionine, glutamine, or arginine;
X28 is glutamine, lysine, asparagine, or arginine; and
X30 is cysteine or is absent (with the proviso that when the amino acid sequence of General Formula 1 is the same as SEQ ID NO: 1, it is excluded).

For example, the glucagon analog may include an amino acid sequence selected from the group consisting of SEQ ID NOS: 2 to 45, and specifically may consist (essentially) of an amino acid sequence selected from the group consisting of SEQ ID NOS: 2 to 45, but is not limited thereto. Further, the glucagon analog may have a pl of 6 to 7, and/or may have *in vitro* activity of 200% or more relative to that of native glucagon. More specifically, the glucagon analog may include an amino acid sequence of SEQ ID NO: 37, and much more specifically, it may consist (essentially) of an amino acid sequence of SEQ ID NO: 37, but is not limited thereto.

Specifically, in General Formula 1,
X1 is histidine, tryptophan, or tyrosine;
X2 is serine or Aib (aminoisobutyric acid);
X7 is cysteine, threonine, or valine;
X10 is tyrosine or cysteine;
X12 is lysine or cysteine;
X13 is tyrosine or cysteine;
X14 is leucine or cysteine;
X15 is aspartic acid or cysteine;
X16 is glutamic acid, serine, or cysteine;
X17 is glutamic acid, lysine, arginine, cysteine, or valine;
X18 is arginine or cysteine;
X19 is alanine or cysteine;
X20 is glutamine or lysine;
X21 is aspartic acid, glutamic acid, valine, or cysteine;
X23 is valine;
X24 is valine or glutamine;
X27 is methionine;
X28 is asparagine or arginine; and
X30 is cysteine or is absent.

For example, the peptide may include an amino acid sequence selected from the group consisting of SEQ ID NOS: 3, 11 to 17, 19 to 27, 29, 31, 33, and 35 to 44, and specifically may consist (essentially) of an amino acid sequence selected from the group consisting of SEQ ID NOS: 3, 11 to 17, 19 to 27, 29, 31, 33, and 35 to 44, but is not limited thereto.

Specifically, in General Formula 1,
X1 is tyrosine;
X2 is Aib (aminoisobutyric acid);
X7 is cysteine, threonine, or valine;
X10 is tyrosine or cysteine;
X12 is lysine;
X13 is tyrosine or cysteine;
X14 is leucine or cysteine;
X15 is aspartic acid or cysteine;
X16 is glutamic acid, serine, or cysteine;
X17 is lysine, arginine, cysteine, or valine;
X18 is arginine or cysteine;
X19 is alanine or cysteine;
X20 is glutamine or lysine;
X21 is aspartic acid, glutamic acid, or cysteine;
X23 is valine;
X24 is glutamine;
X27 is methionine;
X28 is asparagine or arginine; and
X30 is cysteine or is absent (with the proviso that when the amino acid sequence of General Formula 1 is the same as SEQ ID NO: 1, it is excluded).

For example, the peptide may include an amino acid sequence selected from the group consisting of SEQ ID NOS: 12, 14, 17, 19 to 25, 27, 29, 33, 35 to 38, 40 to 42, and 44, and specifically may consist (essentially) of an amino acid sequence selected from the group consisting of SEQ ID NOS: 12, 14, 17, 19 to 25, 27, 29, 33, 35 to 38, 40 to 42, and 44, but is not limited thereto.

Specifically, in General Formula 1,
X1 is histidine, tryptophan, or tyrosine, or is absent;
X2 is serine or Aib (aminoisobutyric acid);
X7 is threonine, valine, or cysteine;
X10 is tyrosine or cysteine;
X12 is lysine or cysteine;
X13 is tyrosine or cysteine;
X14 is leucine or cysteine;
X15 is aspartic acid or cysteine;
X16 is glutamic acid, serine, or cysteine;
X17 is aspartic acid, glutamic acid, lysine, arginine, serine, cysteine, or valine;
X18 is aspartic acid, glutamic acid, arginine, or cysteine;
X19 is alanine or cysteine;
X20 is glutamine, aspartic acid, or lysine;
X21 is aspartic acid or glutamic acid;
X23 is valine;
X24 is valine or glutamine;
X27 is isoleucine or methionine;
X28 is asparagine or arginine; and
X30 is cysteine or is absent (with the proviso that when the amino acid sequence of General Formula 1 is the same as SEQ ID NO: 1, it is excluded).

For example, the peptide may include an amino acid sequence selected from the group consisting of SEQ ID NOS: 2 to 13, 15, 17, 20 to 24, 26 to 30, and 32 to 45, and specifically may consist (essentially) of an amino acid sequence selected from the group consisting of SEQ ID NOS: 2 to 13, 15, 17, 20 to 24, 26 to 30, and 32 to 45, but is not limited thereto.

Specifically, in General Formula 1,
X1 is tyrosine;
X2 is Aib (aminoisobutyric acid);
X7 is threonine;
X10 is tyrosine;
X12 is lysine;
X13 is tyrosine;
X14 is leucine;
X15 is aspartic acid or cysteine;
X16 is glutamic acid, serine, or cysteine;
X17 is lysine or arginine;
X18 is arginine;
X19 is alanine;
X20 is glutamine, cysteine, or lysine;
X21 is aspartic acid, cysteine, valine, or glutamic acid;
X23 is valine or arginine;
X24 is glutamine or leucine;
X27 is methionine;
X28 is asparagine or arginine; and
X30 is absent.

For example, the peptide may include an amino acid sequence selected from the group consisting of SEQ ID NOS: 14, 16, 18, 19, 25, and 31, and specifically may consist (essentially) of an amino acid sequence selected from the group consisting of SEQ ID NOS: 14, 16, 18, 19, 25, and 31, but is not limited thereto.

More specifically, the peptide may be a peptide including an amino acid sequence of the following General Formula 2: 
Y–Aib–QGTF–X7–SD–X10–S–X12–Y–L–X15–X16–X17–R–A–X20–X21–
F–V–X24–W–L–M–N–T–X30 (General Formula 2, SEQ ID NO: 47)
 in General Formula 2,
X7 is threonine, valine, or cysteine;
X10 is tyrosine or cysteine;
X12 is lysine or cysteine;
X15 is aspartic acid or cysteine;
X16 is glutamic acid or serine;
X17 is lysine or arginine;
X20 is glutamine or lysine;
X21 is aspartic acid or glutamic acid;
X24 is valine or glutamine; and
X30 is cysteine or is absent.

For example, the peptide may include an amino acid sequence selected from the group consisting of SEQ ID NOS: 12, 13, 15, and 36 to 44, and specifically may consist (essentially) of an amino acid sequence selected from the group consisting of SEQ ID NOS: 12, 13, 15, and 36 to 44, but is not limited thereto. More specifically, the peptide may include an amino acid sequence of SEQ ID NO: 12, 20, or 37, or may consist (essentially) of the corresponding amino acid sequence, but is not limited thereto.

Specifically, in General Formula 2,
X7 is threonine, valine, or cysteine;
X10 is tyrosine or cysteine;
X12 is lysine;
X15 is aspartic acid;
X16 is glutamic acid or serine;
X17 is lysine or arginine;
X20 is glutamine or lysine;
X21 is aspartic acid or glutamic acid;
X24 is glutamine; and
X30 is cysteine or is absent, but is not particularly limited thereto.

For example, the peptide may include an amino acid sequence selected from the group consisting of SEQ ID NOS: 12, 36 to 38, 40 to 42, and 44, and specifically, may consist (essentially) of an amino acid sequence selected from the group consisting of SEQ ID NOS: 12, 36 to 38, 40 to 42, and 44, but is not limited thereto.

The above-described glucagon analog may include an intramolecular bridge (*e.g.*, covalent crosslinking or non-covalent crosslinking), and specifically, it may be in a form including a ring. For example, the glucagon analog may be in a form where a ring is formed between amino acids at positions 16 and 20 of the glucagon derivative, but it is not particularly limited thereto.

Non-limiting examples of the ring may include a lactam crosslinking (or a lactam ring).

Further, the glucagon analog includes all of those which are modified to include an amino acid capable of forming a ring at the desired site so as to form a ring.

The ring may be formed between side chains of amino acids within the glucagon analog, for example, in the form of a lactam ring formed between a side chain of lysine and a side chain of a glutamic acid, but is not particularly limited thereto.

For example, the peptide including the amino acid sequence of General Formula 1 or 2 may be a peptide in which each amino acid in each amino acid pair among the amino acid pairs of X10 and X14, X12 and X16, X16 and X20, X17 and X21, X20 and X24, and X24 and X28 in General Formula 1 or 2 may be substituted with glutamic acid or lysine, respectively, but is not limited thereto. In Xn (n is a natural number), n refers to the position of the amino acid from the N-terminus of an amino acid sequence provided.

Further, the peptide including the amino acid sequence of General Formula 1 or 2 may be a peptide in which each amino acid in each amino acid pair of X12 and X16, or the amino acid pair of X16 and X20, or the amino acid pair of X17 and X21 is substituted with glutamic acid or lysine, respectively, which is capable of forming a ring, but is not limited thereto.

Further, in General Formula 1 or 2, the peptide may be a peptide in which a ring (*e.g.*, a lactam ring) is formed between respective amino acids in at least one amino acid pair among the amino acid pairs of X10 and X14, X12 and X16, X16 and X20, X17 and X21, X20 and X24, and X24 and X28, but is not limited thereto.

Further, in General Formula 1 or 2, X16 may be glutamic acid, X20 may be lysine, and the side chains of X16 and X20 may form a lactam ring, but they are not limited thereto.

As used herein, the term "glucagon derivative" includes a peptide having one or more differences in the amino acid sequence as compared with native glucagon, a peptide in which the sequence of native glucagon is altered by modification, and a native glucagon mimetic that is able to activate glucagon receptors like native glucagon. Such a derivative of native glucagon may have a blood glucose control function in the living body. In the present invention, the glucagon derivative may be used interchangeably with the glucagon analog.

The glucagon analog or glucagon derivative of the present invention may have improved physical properties by having an altered pl as compared with the native glucagon. Further, the glucagon analog or glucagon derivative may have improved solubility while having the activity of activating glucagon receptors, but is not limited thereto.

Further, the glucagon analog or glucagon derivative may be a glucagon which does not occur naturally.

As used herein, the term "isoelectric point" or "pi" refers to the pH value at which a molecule such as a polypeptide or peptide has no net charge (0). In the case of a polypeptide with various charged functional groups, the net charge thereof is zero at the pl. The net charge of the polypeptide at a pH higher than the pl will be negative, while the net charge of the polypeptide at a pH lower than the pl will be positive.

pl may be determined on an immobilized pH gradient gel consisting of polyacrylamide, starch, or agarose by isoelectric electrophoresis, or may be estimated, for example, from an amino acid sequence using a pl/MW tool (http://expasy.org/tools/pi_tool.html; Gasteiger *et al*., 2003) in an ExPASy server.

As used herein, the term "altered pl" refers to a pl which is different from that of native glucagon due to the substitution of a part of the amino acid sequence of native glucagon with an amino acid residue having a negative charge or a positive charge, i.e., a reduced or increased pl value. The peptide with such an altered pl may exhibit improved solubility and/or high stability at a neutral pH, but is not particularly limited thereto.

More specifically, the glucagon analog or derivative may have an altered pl value, not the pl value (6.8) of native glucagon, and even more specifically a pl value of less than 6.8, more specifically 6.7 or less, more specifically 6.5 or less, and additionally a pl value exceeding 6.8, 7 or higher, more specifically 7.5 or higher, but is not limited thereto, and any pl value different from that of native glucagon will belong to the scope of the present invention. In particular, when the glucagon analog or derivative has a pl value different from that of native glucagon and thus exhibits an improved solubility at neutral pH, as compared with that of native glucagon, thereby showing a low level of aggregation, it is particularly included in the scope of the present invention.

More specifically, the glucagon analog or derivative may have a pl value from 4 to 6.5 and/or from 7 to 9.5, specifically from 7.5 to 9.5, and more specifically from 8.0 to 9.3, but the pl value is not limited thereto. In this case, due to the lower or higher pl value than that of native glucagon, the glucagon analog or derivative may exhibit improved solubility and high stability at neutral pH as compared with native glucagon, but is not particularly limited thereto.

Specifically, the glucagon derivative may be altered by any one method of substitution, addition, deletion, and modification, or a combination thereof in part of the amino acids of native glucagon. The amino acid substitution may be generally made on the basis of similarity in polarity, charge, solubility, hydrophobicity, hydrophilicity, and/or the amphipathic nature of the residues. Conservative substitutions with amino acids having similar properties may be expected to exhibit the same or similar activity.

Examples of the glucagon derivative prepared by way of a combination of the above methods include a peptide having the function of activating a glucagon receptor, which differs in one or more of the amino acid sequences as compared with that of native glucagon, and in which the N-terminal amino acid residue is deaminated, but are not limited thereto, and the derivatives of native glucagon applied in the present invention may be prepared by way of a combination of various methods for preparing the derivatives.

Further, such alteration for the preparation of the derivatives of native glucagon may include all of the modifications using L-type or D-type amino acids, and/or non-native type amino acids; and/or a modification of a native sequence, for example, modification of a side-chain functional group, an intramolecular covalent bonding, e.g., a ring formation between side chains, methylation, acylation, ubiquitination, phosphorylation, aminohexanation, biotinylation, *etc.* Further, the alteration may also include substitutions into non-native compounds.

Further, the alteration may also include all of those where one or more amino acids are added to the amino and/or carboxy terminal of native glucagon.

As the amino acids substituted or added, not only the 20 amino acids commonly found in human proteins, but also atypical amino acids or those which are not naturally occurring may be used. Commercial sources of atypical amino acids may include Sigma-Aldrich, ChemPep Inc., and Genzyme Pharmaceuticals. The peptides including these amino acids and typical peptide sequences may be synthesized and purchased from commercial peptide suppliers, e.g., American Peptide Company, Bachem (USA), or Antigen (Korea).

Amino acid derivatives may also be obtained in the same manner. For example, 4-imidazoacetic acid, *etc.* may be used.

As used herein, the term "fragment of native glucagon, glucagon analog, or glucagon derivative" refers to a form in which one or more amino acids are removed from the amino and/or carboxy terminal of natural glucagon, glucagon analog, or natural glucagon derivative, while having the effect of increasing blood glucose in the body.

Further, the glucagon analog of the present invention may be prepared by using preparation methods used in preparing the derivatives and fragments of natural glucagon, independently or in a combination thereof.

The pharmaceutical composition according to the present invention may include (i) natural glucagon, (ii) a glucagon analog, (iii) a glucagon derivative, (iv) a fragment thereof, or (v) a combination thereof as the glucagon which is one of active ingredients, as long as it has the blood glucose control function of glucagon.

Further, the peptides according to the present invention (e.g., insulin, insulin analog, glucagon, glucagon analog, *etc.*) may include those in which a N-terminus and/or C-terminus are/is not modified. However, modified peptides, in which the N-terminus and/or C-terminus are/is chemically modified or protected by organic groups, or an amino acid is added to the peptide terminus, *etc.,* may also belong to the peptides of the present invention. When the C-terminus is not modified, the terminus of the peptide according to the present invention may have a carboxyl group, but is not particularly limited thereto.

Particularly, in the case of a peptide which is chemically synthesized, its N- and C-termini are charged, and thus N-terminal acetylation and/or C-terminal amidation may be performed to remove the charge, but is not particularly limited thereto.

Specifically, the N- and C-termini of the insulin or the analog thereof, or the glucagon or the analog thereof of the present invention may have an amine group (-NH₂) or a carboxyl group (-COOH), and the C-terminus of the glucagon or the analog thereof may have an amine group, but these are not limited thereto.

Unless specified otherwise in the present invention, the description in the detailed description or claims with respect to the "peptide" according to the present invention or the "conjugate", in which such a peptide is covalently linked to a biocompatible material, may also be applied to the forms which include not only the corresponding peptide or conjugate but also salts of the corresponding peptide or conjugate (e.g., pharmaceutically acceptable salts thereof), or solvates thereof. Accordingly, even in a case where a "peptide" or "conjugate" is described in the present invention, the description may also be equally applied to a particular salt thereof, a particular solvate thereof, and a particular solvate of the particular salt thereof. These salts may be, for example, in a form where any pharmaceutically acceptable salts are used. The kind of the salt is not particularly limited. However, the salt is preferably a salt that is safe and effective to an individual, e.g., a mammal, but is not particularly limited thereto.

The term "pharmaceutically acceptable" refers to a material which may be effectively used for the intended use within the scope of pharmaco-medical decision without inducing excessive toxicity, irritation, allergic responses, *etc.*

As used herein, the term "pharmaceutically acceptable salt" refers to a salt derived from pharmaceutically acceptable inorganic acids, organic acids, or bases. Examples of appropriate acids may include hydrochloric acid, bromic acid, sulfuric acid, nitric acid, perchloric acid, fumaric acid, maleic acid, phosphoric acid, glycolic acid, lactic acid, salicylic acid, succinic acid, toluene-p-sulfonic acid, tartaric acid, acetic acid, citric acid, methanesulfonic acid, formic acid, benzoic acid, malonic acid, naphthalene-2-sulfonic acid, benzenesulfonic acid, *etc.* Examples of the salts derived from appropriate bases may include alkali metals such as sodium, potassium, *etc.*; alkaline earth metals such as magnesium; ammonium, *etc.*

Further, as used herein, the term "solvate" refers to a complex formed between the peptide, conjugate, or salt thereof according to the present invention and a solvent molecule.

Further, the peptide of the present invention may be synthesized according to its length by way of a method well known in the art, e.g., using an automatic peptide synthesizer, and may also be produced by way of genetic engineering technology.

Specifically, the peptide of the present invention may be prepared by way of a standard synthesis method, a recombinant expression system, or any other method known in the art. Accordingly, the glucagon derivative according to the present invention may be synthesized by way of various methods including, for example, the methods described below:
(a) a method of synthesizing a peptide by way of a solid-phase or liquid-phase method stepwise or by fragment assembly, followed by isolation and purification of the final peptide product;
(b) a method of expressing a nucleic acid construct encoding a peptide in a host cell and recovering the expression product from the host cell culture;
(c) a method of performing an *in vitro* cell-free expression of a nucleic acid construct encoding a peptide and recovering the expression product therefrom; or
   a method of obtaining peptide fragments by way of any combination of methods (a), (b), and (c), obtaining a peptide by linking the peptide fragments, and then recovering the peptide.

As a more specific example, a desired peptide may be produced through genetic manipulation by preparing a fusion gene encoding a fusion protein including a fusion partner and insulin or glucagon, or an analog thereof, transforming the fusion gene into a host cell, expressing the fusion gene in the form of a fusion protein, and cleaving and separating insulin or glucagon, or the analog thereof from the fusion protein using a protease or a compound. For this purpose, for example, an amino acid residue-encoding DNA sequence that may be cleaved by a protease such as Factor Xa or enterokinase, CNBr, or a compound such as hydroxylamine, may be inserted between the fusion partner and a polynucleotide encoding insulin or glucagon, or the analog thereof.

In one specific embodiment, the insulin or glucagon of the present invention may be in the form of a long-acting conjugate in which a biocompatible material capable of increasing *in vivo* half-life thereof is linked.

As used herein, the "long-acting conjugate" or "conjugate" of insulin or glucagon has a structure in which insulin or glucagon is linked to a biocompatible material, and may exhibit an enhanced efficacy duration as compared with that of insulin or glucagon which is not linked to the biocompatible material. In the long-acting conjugate, the biocompatible material may be linked to insulin or glucagon via a covalent bond, but is not limited thereto. In the present invention, insulin or glucagon which is one component of the conjugate may be insulin or glucagon having the native sequence, or may be an analog of insulin or glucagon, or a derivative or fragment thereof, in which substitution, addition, deletion, modification, or a combination thereof occurs in one or more amino acids in the native sequence. However, as long as it has the effect of decreasing/increasing blood glucose of natural insulin or glucagon, it may be used as one component of the conjugate of the present invention without limitation.

As used herein, the term "biocompatible material" refers to a material which is linked to a physiologically active material (*e.g.,* a glucagon derivative, an insulinotropic peptide, *etc.*), thereby enhancing the efficacy duration, as compared with a physiologically active material to which a biocompatible material moiety or carrier is not linked. The biocompatible material may be covalently linked to the physiologically active material, but is not particularly limited thereto.

Specifically, the conjugate is a conjugate represented by the following Chemical Formula 1:

### [Chemical Formula 1]

X-La-F

wherein X is the insulin or glucagon;
L is a linker;
a is 0 or a natural number, provided that when a is 2 or more, each L is independent; and
F is a material capable of increasing *in vivo* half-life of X.

The composition of the present invention may include (i) insulin and glucagon, (ii) the long-acting insulin conjugate and glucagon, (iii) insulin and the long-acting glucagon conjugate, or (iv) the long-acting insulin conjugate and the long-acting glucagon conjugate. The insulin or glucagon in the form of the long-acting conjugate may improve side effects of insulin while exhibiting an excellent effect of controlling blood glucose, on the basis of improved efficacy duration.

In the conjugate, F is a material capable of increasing half-life of X, i.e., insulin or glucagon, and corresponds to one constitution of moieties constituting the conjugate of the present invention.

F and X may be linked to each other by a covalent chemical bond or a non-covalent chemical bond, and F and X may be linked to each other via L by a covalent chemical bond, a non-covalent chemical bond, or a combination thereof.

The material capable of increasing half-life of X may be a biocompatible material, and for example, may be selected from the group consisting of a high-molecular-weight polymer, a fatty acid, cholesterol, albumin and a fragment thereof, an albumin-binding material, a polymer of repeating units of a particular amino acid sequence, an antibody, an antibody fragment, an FcRn-binding material, an *in vivo* connective tissue, a nucleotide, fibronectin, transferrin, a saccharide, heparin, and elastin, but is not particularly limited thereto.

The elastin may be human tropoelastin, which is a water-soluble precursor, and may be a polymer of some sequences or some repeating units, for example, including all elastin-like polypeptides, but is not particularly limited thereto.

Examples of the high-molecular-weight polymer may include polymers selected from the group consisting of polyethylene glycol, polypropylene glycol, an ethylene glycol-propylene glycol copolymer, polyoxyethylated polyol, polyvinyl alcohol, polysaccharides, polyvinyl ethyl ether, a biodegradable polymer, a lipid polymer, chitin, hyaluronic acid, oligonucleotides, and combinations thereof, and the polysaccharides may be dextran, but are not particularly limited thereto.

The polyethylene glycol is a general term including all of the forms of homopolymers of ethylene glycol, PEG copolymers, and monomethyl-substituted PEG polymers (mPEG), but is not particularly limited thereto.

Further, the biocompatible material may include poly-amino acids such as poly-lysine, poly-aspartic acid, and poly-glutamic acid, but is not limited thereto.

Further, the fatty acid may have a binding affinity to albumin *in vivo,* but is not particularly limited thereto.

As a more specific example, the FcRn-binding material may be an immunoglobulin Fc region, and more specifically an IgG Fc region, but is not particularly limited thereto.

One or more amino acid side chains within the peptide of the present invention may be attached to the biocompatible material in order to increase in *vivo* solubility and/or half-life, and/or to increase bioavailability thereof. These modifications may reduce the clearance of therapeutic proteins and peptides.

The above-described biocompatible material may be soluble (amphipathic or hydrophilic) and/or non-toxic and/or pharmaceutically acceptable.

F may be directly linked to X (*i.e.,* in Chemical Formula 1, a is 0), or F may be linked to X via a linker (L).

As used herein, the "immunoglobulin Fc region" refers to a region including the heavy chain constant region 2 (CH2) and/or the heavy chain constant region 3 (CH3), excluding the heavy chain and light chain variable regions of an immunoglobulin. The immunoglobulin Fc region may be one constitution that constitutes a moiety of the conjugate of the present invention.

The immunoglobulin Fc region may include a hinge region in the heavy chain constant region, but is not limited thereto. Further, the immunoglobulin Fc region of the present invention may be an extended Fc region including a part or the entirety of the heavy chain constant region 1 (CH1) and/or the light chain constant region 1 (CL1), excluding the heavy chain and the light chain variable regions of the immunoglobulin, as long as the immunoglobulin Fc region has an effect which is substantially the same as or improved compared to the native type. Further, the immunoglobulin Fc region of the present invention may be a region in which a fairly long part of the amino acid sequence corresponding to CH2 and/or CH3 is removed.

For example, the immunoglobulin Fc region of the present invention may be 1) a CH1 domain, a CH2 domain, a CH3 domain, and a CH4 domain; 2) a CH1 domain and a CH2 domain; 3) a CH1 domain and a CH3 domain; 4) a CH2 domain and a CH3 domain; 5) a combination between one or two or more domains among a CH1 domain, a CH2 domain, a CH3 domain, and a CH4 domain and an immunoglobulin hinge region (or a part of the hinge region); and 6) a dimer between each domain of the heavy chain constant region and the light chain constant region, but is not limited thereto.

Further, in one specific embodiment, the immunoglobulin Fc region may be in a dimeric form, and one molecule of X may be covalently linked to an Fc region in a dimeric form, wherein the immunoglobulin Fc and X may be linked to each other via a non-peptide polymer. Furthermore, two molecules of X may also be conjugated in a symmetrical manner to a single Fc region in a dimeric form. In this regard, the immunoglobulin Fc and X may be linked to each other via a non-peptidyl linker, but are not limited to the above-described embodiment.

Further, the immunoglobulin Fc region of the present invention includes not only a native amino acid sequence but also a sequence derivative thereof. An amino acid sequence derivative refers to an amino acid sequence which has a difference in one or more amino acid residues in the native amino acid sequence due to deletion, insertion, non-conservative or conservative substitution, or a combination thereof.

For example, the amino acid residues at positions 214 to 238, 297 to 299, 318 to 322, or 327 to 331, which are known to be important in the binding of IgG Fc, may be used as suitable sites for modification.

Further, various other derivatives are possible, including derivatives that have a deletion of a region capable of forming a disulfide bond, or a deletion of some amino acids at the N-terminus of native Fc, or an addition of a methionine residue at the N-terminus of native Fc. Further, to remove effector functions, a deletion may occur in a complement-binding site, such as a C1q-binding site and an antibody-dependent cell-mediated cytotoxicity (ADCC) site. Techniques of preparing such sequence derivatives of the immunoglobulin Fc region are disclosed in International Patent Publication Nos. WO 97/34631, WO 96/32478, *etc.*

Amino acid exchanges in proteins and peptides which do not generally alter the activity of the molecule are known in the art (H. Neurath, R.L. Hill, The Proteins, Academic Press, New York, 1979). The most commonly occurring exchanges are the amino acid residues Ala/Ser, Val/Ile, Asp/Glu, Thr/Ser, Ala/Gly, Ala/Thr, Ser/Asn, Ala/Val, Ser/Gly, Thy/Phe, Ala/Pro, Lys/Arg, Asp/Asn, Leu/Ile, Leu/Val, Ala/Glu, and Asp/Gly, in both directions. If necessary, the Fc region may be modified by phosphorylation, sulfation, acrylation, glycosylation, methylation, farnesylation, acetylation, amidation, *etc.*

The above-described Fc derivatives exhibit biological activity identical to that of the Fc region of the present invention and have improved structural stability against heat, pH, *etc.*

Further, the Fc region may be obtained from native forms isolated *in vivo* from humans or animals such as cows, goats, pigs, mice, rabbits, hamsters, rats, guinea pigs, *etc.,* or may be recombinants or derivatives thereof obtained from transformed animal cells or microorganisms. Here, a method of obtaining the Fc region from a native immunoglobulin may be a method of isolating a whole immunoglobulin from a living human or animal body, and then treating the isolated immunoglobulin with protease. When treated with papain, the immunoglobulin is cleaved into Fab and Fc regions. When treated with pepsin, the immunoglobulin is cleaved into pF'c and F(ab)₂. Fc or pF'c may be isolated using size-exclusion chromatography, *etc.* In a more specific embodiment, a human-derived Fc region is a recombinant immunoglobulin Fc region obtained from a microorganism.

In addition, the immunoglobulin Fc region may have natural sugar chains, or increased or decreased sugar chains as compared with the natural type, or may be in a deglycosylated form. The increase, decrease, or removal of the sugar chains of the immunoglobulin Fc may be achieved by way of common methods such as a chemical method, an enzymatic method, and a genetic engineering method using a microorganism. Here, the immunoglobulin Fc region obtained by removal of sugar chains from the Fc region exhibits a significant decrease in binding affinity to the C1q part, and a decrease or loss in antibody-dependent cytotoxicity or complement-dependent cytotoxicity, and thus it does not induce unnecessary immune responses *in vivo.* In this aspect, a deglycosylated or aglycosylated immunoglobulin Fc region may be a more suitable form to meet the original object of the present invention as a drug carrier.

As used herein, the term "deglycosylation" refers to enzymatically removing sugar moieties from an Fc region, and the term "aglycosylation" refers to an unglycosylated Fc region produced in prokaryotes, more specifically, *E. coli.*

Meanwhile, the immunoglobulin Fc region may be derived from humans or other animals including cows, goats, pigs, mice, rabbits, hamsters, rats, and guinea pigs. In a more specific embodiment, it is derived from humans.

Further, the immunoglobulin Fc region may be derived from IgG, IgA, IgD, IgE, IgM, or a combination or hybrid thereof. In a more specific embodiment, it is derived from IgG or IgM, which are the most abundant proteins in human blood, and in an even more specific embodiment, it is derived from IgG, which is known to enhance the half-lives of ligand-binding proteins. In a yet even more specific embodiment, the immunoglobulin Fc region is an IgG4 Fc region, and in the most specific embodiment, the immunoglobulin Fc region is an aglycosylated Fc region derived from human IgG4, but is not limited thereto.

Meanwhile, as used herein, the "combination" means that polypeptides encoding single-chain immunoglobulin Fc regions of the same origin are linked to a single-chain polypeptide of a different origin to form a dimer or multimer. That is, a dimer or multimer may be formed from two or more fragments selected from the group consisting of IgG Fc, IgA Fc, IgM Fc, IgD Fc, and IgE Fc fragments.

Further, the above-described conjugate may have increased efficacy of duration, as compared with natural insulin or glucagon or as compared with X not modified with F, and the conjugate includes not only the above-described form but also a form in which the conjugate is included in biodegradable nanoparticles.

Further, L is a peptidyl linker or a non-peptidyl linker.

When L is a peptidyl linker, it may include one or more amino acids, for example, 1 amino acid to 1000 amino acids, but is not particularly limited thereto. In the present invention, to link F and X, a variety of known peptidyl linkers may be used, and examples thereof may include a [GS]ₓ linker, a [GGGS]X linker, and a [GGGGS]ₓ linker, wherein x may be a natural number of 1 or more, but the linkage is not limited thereto.

As used herein, the "non-peptidyl linker" includes a biocompatible polymer in which two or more repeating units are linked. The repeating units are linked to each other through any covalent bond, not a peptide bond. The non-peptidyl linker may be one constitution that constitutes a moiety of the conjugate of the present invention, and corresponds to L in Chemical Formula 1. In the present invention, the non-peptidyl linker may be used interchangeably with a "non-peptidyl polymer".

In the present invention, the non-peptidyl linker includes reactive groups at its ends, and thus a conjugate may be formed by reacting with other components which constitute the conjugate. When the non-peptidyl polymer having reactive functional groups at both ends may be linked to X and F in Chemical Formula 1 through the reactive groups to form the conjugate, the non-peptidyl linker or non-peptidyl polymer may be named as a non-peptidyl polymer linker moiety or non-peptidyl linker moiety.

In Lₐ, a may be 1 or more, and when a is 2 or more, each L may be independent.

Further, in one specific embodiment, the conjugate may be a conjugate in which F, specifically the immunoglobulin Fc region, and X, specifically the peptide drug, may be covalently linked to each other via a non-peptidyl linker including reactive groups capable of reacting with X and F.

Specifically, the non-peptidyl linker may be selected from the group consisting of fatty acids, polysaccharides, high-molecular-weight polymers, low-molecular-weight compounds, nucleotides, and combinations thereof.

In the present invention, the high-molecular-weight polymer may have a molecular weight in the range of more than 0 kDa and about 100 kDa, specifically about 1 kDa to about 100 kDa, and more specifically about 1 kDa to about 20 kDa, but is not particularly limited thereto.

As used herein, the term "about" refers to a range including all of ±0.5, ±0.4, ±0.3, ±0.2, ±0.1, *etc.,* and it includes all of the values equivalent to those which come immediately after the term "above" or those in a similar range, but is not limited thereto.

The polymer may include, but is not particularly limited to, polymers selected from the group consisting of polyethylene glycol, polypropylene glycol, an ethylene glycol-propylene glycol copolymer, polyoxyethylated polyol, polyvinyl alcohol, polysaccharides, polyvinyl ethyl ether, a biodegradable polymer, a lipid polymer, chitin, hyaluronic acid, oligonucleotides, and combinations thereof, and the polysaccharides may be dextran. In a more specific embodiment, L may be polyethylene glycol, but is not limited thereto. Further derivatives thereof which are already known in the art, and derivatives which may be easily prepared at the level of the technology in the art may also be included in the scope of the present invention.

The non-peptidyl linker which may be used in the present invention may be used without limitation, as long as it is a polymer which has resistance to *in vivo* proteases. The molecular weight of the non-peptidyl polymer may be in the range of about 1 kDa to about 100 kDa, and specifically about 1 kDa to about 20 kDa, but is not limited thereto. Further, the non-peptidyl linker of the present invention, which is linked to the polypeptide corresponding to F, may include not only a single kind of a polymer but also a combination of different kinds of polymers.

As used herein, the term "about" refers to a range including all of ±0.5, ±0.4, ±0.3, ±0.2, ±0.1, *etc.,* and it includes all of the values equivalent to those which come immediately after the term "above" or those in a similar range, but is not limited thereto.

In one specific embodiment, both ends of the non-peptidyl linker may be linked to an amine group or a thiol group of F, i.e., the immunoglobulin Fc region, and an amine group or a thiol group of X.

Specifically, the non-peptidyl polymer may include reactive groups at both ends, the reactive groups capable of binding to F (i.e., immunoglobulin Fc region) and X, respectively, and specifically, reactive groups capable of binding to an amine group located at the N-terminus or lysine, or a thiol group of cysteine of X or F (e.g., immunoglobulin Fc region), but is not limited thereto.

Further, the reactive groups of the non-peptidyl polymer, which are able to bind to F, e.g., immunoglobulin Fc region, and X, may be selected from the group consisting of an aldehyde group, a maleimide group, and a succinimide derivative, but are not limited thereto.

In the above, the aldehyde group may be exemplified by a propionaldehyde group or a butyraldehyde group, but is not limited thereto.

In the above, the succinimide derivative may be exemplified by succinimidyl valerate, succinimidyl methylbutanoate, succinimidyl methylpropionate, succinimidyl butanoate, succinimidyl propionate, *N-*hydroxysuccinimide, hydroxy succinimidyl, succinimidyl carboxymethyl, or succinimidyl carbonate, but is not limited thereto.

The non-peptidyl linker may be linked to X and F via these reactive groups, but is not particularly limited thereto.

Further, the final product produced through reductive amination via an aldehyde bond is more stable than that linked by an amide bond. The aldehyde reactive group selectively reacts with the N-terminus at a low pH while it may form a covalent bond with a lysine residue at high pH, e.g., pH 9.0.

Further, the reactive groups at both ends of the non-peptidyl linker may be the same as or different from each other; for example, a maleimide group may be provided at one end, and an aldehyde group, a propionaldehyde group, or a butyraldehyde group may be provided at the other end. However, if F, specifically an immunoglobulin Fc region, and X may be conjugated at each end of the non-peptidyl linker, the reactive groups are not particularly limited.

For example, the non-peptidyl polymer may include, as reactive groups, a maleimide group at one end and an aldehyde group, a propionaldehyde group, or a butyraldehyde group at the other end.

When polyethylene glycol having a reactive hydroxy group at both ends thereof is used as the non-peptidyl polymer, the hydroxy group may be activated to various reactive groups by known chemical reactions, or polyethylene glycol having a commercially available modified reactive group may be used so as to prepare the long-acting protein conjugate of the present invention.

In a specific embodiment, the non-peptidyl polymer may be linked to a cysteine residue of X, and more specifically to the -SH group of cysteine, but is not limited thereto.

For example, the non-peptidyl polymer may be linked to a cysteine residue at position 10, a cysteine residue at position 13, a cysteine residue at position 15, a cysteine residue at position 17, a cysteine residue at position 19, a cysteine residue at position 21, a cysteine residue at position 24, a cysteine residue at position 28, a cysteine residue at position 29, a cysteine residue at position 30, a cysteine residue at position 31, a cysteine residue at position 40, or a cysteine residue at position 41 in the peptide corresponding to X, but is not particularly limited thereto.

Specifically, the reactive group of the non-peptidyl polymer may be linked to the -SH group of the cysteine residue, and the above description also applies to the reactive group. When maleimide-PEG-aldehyde is used, the maleimide group may be linked to the -SH group of X by a thioether bond, and the aldehyde group may be linked to -NH₂ of F, specifically the immunoglobulin Fc, through reductive amination, but is not limited thereto, and the above is merely an exemplary embodiment.

Further, in the above conjugate, the reactive group of the non-peptidyl polymer may be linked to NH₂ located at the N-terminus of the immunoglobulin Fc region, but this is merely an exemplary embodiment.

As used herein, the term "prevention" refers to all kinds of actions associated with inhibition or delay of the occurrence of insulin-related diseases by administering the composition, and the term "treatment" refers to all kinds of actions associated with the improvement or advantageous changes in symptoms of insulin-related diseases due to administration of the composition.

As used herein, the term "administration" refers to an introduction of a particular material to a patient in an appropriate manner. The composition may be administered via a general route that enables the delivery of the composition to a target tissue *in vivo,* for example, intraperitoneal, intravenous, intramuscular, subcutaneous, intradermal, oral, topical, intranasal, intrapulmonary, and intrarectal administration, but is not particularly limited thereto.

The pharmaceutical composition according to the present invention may include a pharmaceutically acceptable carrier.

As used herein, the term "pharmaceutically acceptable" refers to the properties of having a sufficient amount to exhibit a therapeutic effect and not causing adverse effects, and may be easily determined by a skilled person in the art based on the factors well known in the medical field, such as the kind of disease, a patient's age, body weight, health status, sex, drug sensitivity, administration route, administration method, administration frequency, duration of treatment, a drug to be mixed or administered simultaneously in combination, *etc.*

The pharmaceutically acceptable carrier may include, for oral administration, a binder, a glidant, a disintegrant, an excipient, a solubilizing agent, a dispersant, a stabilizing agent, a suspending agent, a coloring agent, a flavoring agent, *etc.*; for injections, a buffering agent, a preserving agent, an analgesic, a solubilizing agent, an isotonic agent, a stabilizing agent, *etc.,* which may be used in combination; and for topical administrations, a base, an excipient, a lubricant, a preserving agent, *etc.* The formulation of the pharmaceutical composition of the present invention may be prepared variously by combining with a pharmaceutically acceptable carrier as described above. For example, for oral administration, the composition may be formulated into tablets, troches, capsules, elixirs, suspensions, syrups, wafers, *etc.* For injections, the composition may be formulated into single-dose ampoules or multi-dose containers. The composition may be also formulated into solutions, suspensions, tablets, pills, capsules, sustained-release formulations, *etc.*

Meanwhile, examples of carriers, excipients, and diluents suitable for formulation may include lactose, dextrose, sucrose, sorbitol, mannitol, xylitol, erythritol, maltitol, starch, acacia rubber, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, mineral oil, *etc.* Additionally, the composition may further include a filler, an anti-coagulant, a lubricant, a humectant, a flavoring agent, an emulsifier, a preservative, *etc.*

Further, the pharmaceutical composition of the present invention may be prepared in any formulation type selected from the group consisting of tablets, pills, powders, granules, capsules, suspensions, liquid medicine for internal use, emulsions, syrups, sterile aqueous solutions, non-aqueous solvents, lyophilized formulations, and suppositories.

Further, the conjugate may be used by blending with various pharmaceutically acceptable carriers such as physiological saline or organic solvents. In order to increase the stability or absorptivity, carbohydrates such as glucose, sucrose, or dextrans; antioxidants such as ascorbic acid or glutathione; chelating agents; low-molecular-weight proteins; or other stabilizers may be used.

The administration dose and frequency of the pharmaceutical composition of the present invention are determined by the type of active ingredient(s), along with various factors, such as the disease to be treated, administration route, a patient's age, sex, and body weight, and severity of the disease.

The total effective dose of the composition of the present invention may be administered to a patient in a single dose, or may be administered for a long period of time in multiple doses according to a fractionated treatment protocol. In the pharmaceutical composition of the present invention, the content of active ingredient(s) may vary depending on the disease severity. Specifically, the preferable total daily dose of the conjugate of the present invention may be approximately 0.0001 mg to 500 mg per 1 kg of body weight of a patient. However, the effective dose of the conjugate is determined considering various factors including a patient's age, body weight, health conditions, sex, disease severity, diet, and excretion rate, in addition to administration route and treatment frequency of the pharmaceutical composition. In this regard, those skilled in the art may easily determine the effective dose suitable for the particular use of the composition of the present invention. The pharmaceutical composition according to the present invention is not particularly limited to the formulation and administration route and mode, as long as it shows the effects of the present invention.

With regard to the pharmaceutical composition of the present invention, the insulin or the long-acting conjugate thereof and the glucagon or the long-acting conjugate thereof may be administered simultaneously, sequentially, or in reverse order, but the administration is not limited thereto.

As described above, the combined administration may include
a) administration of (i) the insulin or the long-acting conjugate thereof and (ii) the glucagon or the long-acting conjugate thereof in a single mixture; or
b) administration of (i) the insulin or the long-acting conjugate thereof and (ii) the glucagon or the long-acting conjugate thereof each in a separate form, but is not limited thereto.

When the insulin or the long-acting conjugate thereof and the glucagon or the long-acting conjugate thereof are each administered in a separate form, the insulin or the long-acting conjugate thereof and the glucagon or the long-acting conjugate thereof are formulated into separate preparations, which are then administered simultaneously, individually, sequentially, or in reverse order.

To achieve the present invention, still another aspect provides a pharmaceutical composition for preventing or treating hypoglycemia, the pharmaceutical composition including insulin and glucagon.

Specifically, the composition may include
(i) insulin and glucagon;
(ii) a long-acting insulin conjugate, in which insulin and a biocompatible material capable of increasing *in vivo* half-life thereof are linked to each other, and glucagon;
(iii) insulin and a long-acting glucagon conjugate, in which glucagon and a biocompatible material capable of increasing *in vivo* half-life thereof are linked to each other; or
(iv) the long-acting insulin conjugate and the long-acting glucagon conjugate.

The insulin or glucagon of the present invention may be insulin or glucagon having a native sequence, or may be an analog, derivative, or fragment thereof which is obtained by introducing an alteration of substitution, addition, deletion, modification, or a combination thereof in one or more amino acids, as described above.

The pharmaceutical composition for preventing or treating hypoglycemia according to the present invention may include the insulin or the long-acting conjugate thereof and the glucagon or the long-acting conjugate thereof, and thus may have the effect of alleviating hypoglycemia which occurs as a side effect caused by insulin administration while having the effect of suppressing weight gain.

In one exemplary embodiment of the present invention, blood glucose-lowering, weight gain-suppressing, and hypoglycemia-alleviating effects were confirmed in db/db mice administered with the composition including the insulin or the long-acting conjugate thereof and the glucagon or the long-acting conjugate thereof, and thus it was demonstrated that the pharmaceutical composition according to the present invention may maintain insulin efficacy while improving side effects thereof.

With regard to the pharmaceutical composition of the present invention, the insulin or the long-acting conjugate thereof and the glucagon or the long-acting conjugate thereof may be administered in combination simultaneously, sequentially, or in reverse order, but are not limited thereto. The combined administration is the same as described above.

To achieve the present invention, still another aspect provides a composition for improving side effects of insulin, the composition including insulin and glucagon.

Specifically, the composition may include
(i) insulin and glucagon;
(ii) a long-acting insulin conjugate, in which insulin and a biocompatible material capable of increasing *in vivo* half-life thereof are linked to each other, and glucagon;
(iii) insulin and a long-acting glucagon conjugate, in which glucagon and a biocompatible material capable of increasing *in vivo* half-life thereof are linked to each other; or
(iv) the long-acting insulin conjugate and the long-acting glucagon conjugate.

The insulin or glucagon of the present invention may be insulin or glucagon having a native sequence, or may be an analog, derivative, or fragment thereof which is obtained by introducing an alteration of substitution, addition, deletion, modification, or a combination thereof in one or more amino acids, as described above.

The composition for improving side effects of insulin according to the present invention may include the insulin or the long-acting conjugate thereof and the glucagon or the long-acting conjugate thereof, and thus may exhibit the effect of alleviating side effects caused by insulin administration (e.g., hypoglycemia or weight gain).

With regard to the pharmaceutical composition of the present invention, the insulin or the long-acting conjugate thereof and the glucagon or the long-acting conjugate thereof may be administered in combination simultaneously, sequentially, or in reverse order, but are not limited thereto. The combined administration is the same as described above.

To achieve the present invention, still another aspect provides a complex formulation for improving hypoglycemia of a patient with an insulin-related disease, the complex formulation including:
(i) insulin and glucagon;
(ii) a long-acting insulin conjugate and glucagon;
(iii) insulin and a long-acting glucagon conjugate; or
(iv) the long-acting insulin conjugate and the long-acting glucagon conjugate.

In one specific embodiment, the complex formulation according to the present invention may include a pharmaceutically acceptable carrier.

As used herein, the terms "pharmaceutically acceptable" and "pharmaceutically acceptable carrier" are the same as described above.

The complex formulation according to the present invention may be a complex formulation for treating an insulin-related disease, and may exhibit the effect of alleviating side effects caused by insulin administration (e.g., hypoglycemia or weight gain). Specifically, the complex formulation may improve hypoglycemia, which is a side effect of insulin, and may also suppress weight gain.

The complex formulation of the present invention may include the insulin or the long-acting conjugate thereof and the glucagon or the long-acting conjugate thereof at a weight ratio of 0.1: 1 to 100:1, or at a molar ratio of 1:1 to 30:1, 1:1 to 20:1, or 1:1 to 16:1, but is not limited thereto.

To achieve the present invention, still another aspect provides a method of preventing or treating an insulin-related disease, the method including the step of administering (i) insulin and glucagon; (ii) a long-acting insulin conjugate and glucagon; (iii) insulin and a long-acting glucagon conjugate; or (iv) the long-acting insulin conjugate and the long-acting glucagon conjugate to an individual who is at risk of developing the insulin-related disease or has the insulin-related disease.

The step of administering may be performed by combined administration of (i) insulin and glucagon, (ii) the long-acting insulin conjugate and glucagon, (iii) insulin and the long-acting glucagon conjugate, or (iv) the long-acting insulin conjugate and the long-acting glucagon conjugate. The substances may be administered simultaneously, sequentially, or in reverse order, and co-administered in a combination of appropriate effective amounts, but is not limited to a particular administration method or order. Further, the prevention or treatment method of the present invention may be administering, to an individual, the composition or complex formulation including any one of (i) to (iv), but is not limited thereto. The insulin or the long-acting conjugate thereof and the glucagon or the long-acting conjugate thereof may be individual formulations or a complex formulation.

The composition or the complex formulation including the insulin or the long-acting conjugate thereof and the glucagon or the long-acting conjugate thereof of the present invention may supplement the activity or function of insulin to significantly lower the blood glucose while suppressing the side effects of insulin such as weight gain and hypoglycemia, thereby showing excellent therapeutic effects on insulin-related diseases.

Further, the composition or the complex formulation may be formulated into a single-dosage form suitable for a patient's body, and specifically into a preparation useful for administration of protein drugs according to a common method in the pharmaceutical field, and may be administered using an administration method commonly used in the art via an oral or parenteral route, such as through skin, intravenously, intramuscularly, intra-arterially, intramedullarily, intrathecally, intraventricularly, pulmonarily, transdermally, subcutaneously, intraperitoneally, intranasally, intragastrically, topically, sublingually, vaginally, or rectally, but is not limited thereto.

The method of the present invention may include administering the pharmaceutical composition including the peptide in a pharmaceutically effective amount. The total daily dose should be determined within appropriate medical judgment by a physician and administered once or several times in divided doses. With respect to the objects of the present invention, the specific therapeutically effective dose for any particular patient may be preferably applied differently depending on various factors well known in the medical art, including the kind and degree of the response to be achieved, specific compositions including whether other agents are occasionally used therewith or not, a patient's age, body weight, general health conditions, sex and diet, the time and route of administration, secretion rate of the composition, duration of treatment, other drugs used in combination or concurrently with the specific composition, and similar factors well known in the medical arts.

To achieve the present invention, still another aspect provides a method of improving side effects of insulin, the method including the step of administering (i) insulin and glucagon; (ii) a long-acting insulin conjugate and glucagon; (iii) insulin and a long-acting glucagon conjugate; or (iv) the long-acting insulin conjugate and the long-acting glucagon conjugate to an individual who is at risk of developing the insulin-related disease or has the insulin-related disease.

Still another aspect of the present invention provides use of the composition or the complex formulation in preparing a medicament for treating an insulin-related disease.

### [Mode for Invention]

Hereinafter, the present invention will be described in more detail with reference to the following Examples. However, the following Examples are for illustrative purposes only, and the scope of the present invention is not limited thereto.

### Example 1: Preparation of Glucagon Analogs

The present inventors intended to confirm therapeutic effects on diabetes according to combined administration of insulin and glucagon, and furthermore to determine whether the risk of hypoglycemia, which is a side effect of insulin, may be reduced due to the combined administration.

Accordingly, a native glucagon and glucagon analogs having the equivalent activity thereto were prepared as described in Table 1 below. The glucagon analogs were synthesized with reference to Korean Patent Publication Nos. 10-2016-0082482, 10-2017-003466, and 10-2018-0002544, and pl and *in vitro* activity thereof were measured.

**[Table 1]**

| SEQ ID NO: | Glucagon analog sequence | Ring formation | pl | *In vitro* activity (relative activity to SEQ ID NO: 1, %) |
|---|---|---|---|---|
| SEQ ID NO: 1 | HSQGTFTSDYSKYLDSRRAQDFVQWLMNT | - | 6.8 | 100 |
| SEQ ID NO: 2 | HSQGTFTSDYSKYLDCDRAQDFVQWLMNT | - | 4.56 | 0.6 |
| SEQ ID NO: 3 | HSQGTFTSDYSKYLDCERAQDFVQWLMNT | - | 4.66 | 6.1 |
| SEQ ID NO: 4 | HSQGTFTSDYSKYLDSCDAQDFVQWLMNT | - | 4.13 | < 0.1 |
| SEQ ID NO: 5 | HSQGTFTSDYSKYLDSCEAQDFVQWLMNT | - | 4.22 | 0.3 |
| SEQ ID NO: 6 | HSQGTFTSDYSKYLDSCEADDFVQWLMNT | - | 4.03 | < 0.1 |
| SEQ ID NO: 7 | YSQGTFTSDYSKYLDSCEADDFVQWLMNT | - | 3.71 | < 0.1 |
| SEQ ID NO: 8 | YXQGTFTSDYSKYLDSCDAQDFVQWLINT | - | 3.77 | < 0.1 |
| SEQ ID NO: 9 | YXQGTFTSDYSKYLDSCDAQDFVVWLINT | - | 3.77 | < 0.1 |
| SEQ ID NO: 10 | YXQGTFTSDYSKYLDSCDADDFVVWLINT | - | 3.66 | < 0.1 |
| SEQ ID NO: 11 | YXQGTFTSDYSKYLDEKCAKEFVQWLMNT | - | 4.78 | 4.6 |
| SEQ ID NO: 12 | YXQGTFTSDYSKYLDEKRAKEFVQWLMNTC | Ring formed | 6.20 | 56.3 |
| SEQ ID NO: 13 | YXQGTFTSDYSCYLDSRRAQDFVQWLMNT | - | 4.43 | 5.2 |
| SEQ ID NO: 14 | YXQGTFTSDYSKYLDCKRAKEFVQWLMNT | - | 8.12 | 18.1 |
| SEQ ID NO: 15 | YXQGTFTSDYSKYLCEKRAQDFVVWLMNT | - | 6.11 | 1.1 |
| SEQ ID NO: 16 | YXQGTFTSDYSKYLDCRRAQVFVQWLMRT | - | 9.11 | 4.2 |
| SEQ ID NO: 17 | YXQGTFTSDYSKYLDCVRAQDFVQWLMRT | - | 6.03 | 23.2 |
| SEQ ID NO: 18 | YXQGTFTSDYSKYLDSRRACDFRLWLMNT | - | 8.15 | < 0.1 |
| SEQ ID NO: 19 | YXQGTFTSDYSKYLC**E**KRA**K**EFVQWLMNT | Ring formed | 8.12 | 12.1 |
| SEQ ID NO: 20 | YXQGTFTSDYSKYLD**E**CRA**K**EFVQWLMNT | Ring formed | 4.78 | 299.7 |
| SEQ ID NO: 21 | YXQGTFTSDYSKYLD**E**KCA**K**EFVQWLMNT | Ring formed | 4.78 | 57.8 |
| SEQ ID NO: 22 | YXQGTFTSDYSKYLD**E**KRC**K**EFVQWLMNT | Ring formed | 6.20 | 147.8 |
| SEQ ID NO: 23 | YXQGTFTSDYSKYCD**E**KRA**K**EFVQWLMNT | Ring formed | 6.20 | 76.8 |
| SEQ ID NO: 24 | YXQGTFTSDYSKCLD**E**KRA**K**EFVQWLMNT | Ring formed | 6.21 | 58.0 |
| SEQ ID NO: 25 | YXQGTFTSDYSKYLD**E**KRA**K**CFVQWLMNT | Ring formed | 8.12 | 46.9 |
| SEQ ID NO: 26 | WXQGTFTSDYSKYLD**E**CRA**K**DFVQWLMNT | Ring formed | 4.68 | 1.0 |
| SEQ ID NO: 27 | YXQGTFVSDYSKYLD**E**CRA**K**DFVQWLMNT | Ring formed | 4.68 | 93.6 |
| SEQ ID NO: 28 | WXQGTFVSDYSKYLD**E**CRA**K**DFVQWLMNT | Ring formed | 4.68 | < 0.1 |
| SEQ ID NO: 29 | YXQGTFTSDYSKCLD**E**RRA**K**DFVQWLMNT | Ring formed | 6.15 | 61.3 |
| SEQ ID NO: 30 | WXQGTFTSDYSKCLD**E**RRA**K**DFVQWLMNT | Ring formed | 4.44 | 0.3 |
| SEQ ID NO: 31 | YXQGTFTSDYSKYLDC**K**RAK**E**FVQWLMNT | Ring formed | 8.12 | 6.3 |
| SEQ ID NO: 32 | -SQGTFTSDYSKYLD**E**CRA**K**EFVQWLMNT | Ring formed | 4.78 | 0.7 |
| SEQ ID NO: 33 | YXQGTFTSDYSKYLDSRRAQDFVQWLMNT | - | 6.04 | 108.2 |
| SEQ ID NO: 34 | WXQGTFTSDYSKYCD**E**RRA**K**EFVQWLMNT | Ring formed | 6.21 | 0.2 |
| SEQ ID NO: 35 | YXQGTFTSDYSKYCD**E**RRA**K**EFVQWLMNT | Ring formed | 6.2 | 17.7 |
| SEQ ID NO: 36 | YXQGTFTSDCSKYLD**E**RRA**K**EFVQWLMNT | Ring formed | 6.21 | 9.9 |
| SEQ ID NO: 37 | YXQGTFTSDYSKYLD**E**RRA**K**EFVQWLMNTC | Ring formed | 6.21 | 225.5 |
| SEQ ID NO: 38 | YXQGTFCSDYSKYLD**E**RRA**K**EFVQWLMNT | Ring formed | 6.15 | 167.3 |
| SEQ ID NO: 39 | YXQGTFVSDCSKYLD**E**RRA**K**DFVQWLMNT | Ring formed | 6.15 | 3.7 |
| SEQ ID NO: 40 | YXQGTFVSDYSKYLD**E**RRA**K**DFVQWLMNT C | Ring formed | 6.15 | 40.8 |
| SEQ ID NO: 41 | YXQGTFCSDYSKYLD**E**RRA**K**DFVQWLMNT | Ring formed | 6.03 | 45.2 |
| SEQ ID NO: 42 | YXQGTFCSDYSKYLDSRRAQDFVQWLMNT | - | 6.03 | 37.9 |
| SEQ ID NO: 43 | YXQGTFTSDCSKYLDSRRAQDFVQWLMNT | - | 6.03 | 1.6 |
| SEQ ID NO: 44 | YXQGTFTSDYSKYLDSRRAQDFVQWLMNT C | - | 6.21 | 75.4 |
| SEQ ID NO 45 | YXQGTFTSDYSCYLDEKRAKEFVQWLMNT | - | 4.78 | |

In the sequences described in Table 1, the amino acid represented by X represents a non-native amino acid, aminoisobutyric acid (Aib), the underlined amino acid residues represent formation of a lactam ring between the side chains of the corresponding underlined amino acids, and "-" indicates that no amino acid residue is present on the corresponding position. Further, in the rows with regard to ring formation, "-" indicates that there is no ring formation in the corresponding sequences.

### Example 2: Preparation of Insulin Analogs

The present inventors intended to prepare insulin analogs for combined administration with glucagon or a long-acting conjugate thereof. Specifically, analogs of Table 2 below were prepared with reference to Korean Patent Publication No. 10-2014-0106452.

Alteration in the amino acid sequence of an A-chain or a B-chain and names of analogs are shown in Table 2 below. That is, analog 1 indicates that glycine at position 1 of the A-chain was substituted with alanine, and analog 4 indicates that glycine at position 8 of the B-chain was substituted with alanine.

**[Table 2]**

| Analog | Amino acid alteration |
|---|---|
| Analog 1 | A¹G -> A |
| Analog 2 | A²I->A |
| Analog 3 | A¹⁹Y -> A |
| Analog 4 | B⁸G -> A |
| Analog 5 | B²³G -> A |
| Analog 6 | B²⁴F -> A |
| Analog 7 | B²⁵F -> A |
| Analog 8 | A¹⁴Y -> E |
| Analog 9 | A¹⁴Y -> N |

DNA sequences and protein sequences of each of insulin analogs 1 to 9 are shown in Table 3 below.

**[Table 3]**

| | Sequence | | SEQ ID NO: |
|---|---|---|---|
| Analog 1 | DNA | ttc gtt aac caa cac ttg tgt ggc tca cac ctg gtg gaa gct ctc tac cta [?]0 gtg tgc ggg gaa cga ggc ttc ttc tac aca ccc aag acc cgc cgg gag gca gag gac ctg cag gtg ggg cag gtg gag ctg ggc ggg ggc cct ggt gca ggc agc ctg cag ccc ttg gcc ctg gag ggg tcc ctg cag aag cgt gcg att gtg gaa caa tgc tgt acc agc atc tgc tcc ctc tac cag ctg gag aac tac tgc aac | 50 |
| | Protein | Phe Val Asn Gln His Leu Cys Gly Ser His Leu Val Glu Ala Leu Tyr 51 Leu Val Cys Gly Glu Arg Gly Phe Phe Tyr Thr Pro Lys Thr Arg Arg Glu Ala Glu Asp Leu Gln Val Gly Gln Val Glu Leu Gly Gly Gly Pro Gly Ala Gly Ser Leu Gln Pro Leu Ala Leu Glu Gly Ser Leu Gln Lys Arg Ala Ile Val Glu Gln Cys Cys Thr Ser Ile Cys Ser Leu Tyr Gln | 51 |
| | | Leu Glu Asn Tyr Cys Asn | |
| Analog 2 | DNA | ttc gtt aac caa cac ttg tgt ggc tca cac ctg gtg gaa gct ctc tac cta 52 gtg tgc ggg gaa cga ggc ttc ttc tac aca ccc aag acc cgc cgg gag gca gag gac ctg cag gtg ggg cag gtg gag ctg ggc ggg ggc cct ggt gca ggc agc ctg cag ccc ttg gcc ctg gag ggg tcc ctg cag aag cgt ggc gcg gtg gaa caa tgc tgt acc agc atc tgc tcc ctc tac cag ctg gag aac tac tgc aac | 52 |
| | Protein | Phe Val Asn Gln His Leu Cys Gly Ser His Leu Val Glu Ala Leu Tyr 53 Leu Val Cys Gly Glu Arg Gly Phe Phe Tyr Thr Pro Lys Thr Arg Arg Glu Ala Glu Asp Leu Gln Val Gly Gln Val Glu Leu Gly Gly Gly Pro Gly Ala Gly Ser Leu Gln Pro Leu Ala Leu Glu Gly Ser Leu Gln Lys Arg Gly Ala Val Glu Gln Cys Cys Thr Ser Ile Cys Ser Leu Tyr Gln Leu Glu Asn Tyr Cys Asn | 53 |
| Analog 3 | DNA | | 54 |
| | | gca gag gac ctg cag gtg ggg cag gtg gag ctg ggc ggg ggc cct ggt gca ggc agc ctg cag ccc ttg gcc ctg gag ggg tcc ctg cag aag cgt ggc att gtg gaa caa tgc tgt acc agc atc tgc tcc ctc tac cag ctg gag aac gcg tgc aac | |
| | Protein | Phe Val Asn Gln His Leu Cys Gly Ser His Leu Val Glu Ala Leu Tyr 55 Leu Val Cys Gly Glu Arg Gly Phe Phe Tyr Thr Pro Lys Thr Arg Arg Glu Ala Glu Asp Leu Gln Val Gly Gln Val Glu Leu Gly Gly Gly Pro Gly Ala Gly Ser Leu Gln Pro Leu Ala Leu Glu Gly Ser Leu Gln Lys Arg Gly Ile Val Glu Gln Cys Cys Thr Ser Ile Cys Ser Leu Tyr Gln Leu Glu Asn Ala Cys Asn | 55 |
| Analog 4 | DNA | ttc gtt aac caa cac ttg tgt gcg tca cac ctg gtg gaa gct ctc tac cta 56 gtg tgc ggg gaa cga ggc ttc ttc tac aca ccc aag acc cgc cgg gag gca gag gac ctg cag gtg ggg cag gtg gag ctg ggc ggg ggc cct ggt gca ggc agc ctg cag ccc ttg gcc ctg gag ggg tcc ctg cag aag cgt ggc att gtg gaa caa tgc tgt acc agc atc tgc tcc ctc tac cag ctg gag | 56 |
| | | aac tac tgc aac | |
| | Protein | Phe Val Asn Gln His Leu Cys Ala Ser His Leu Val Glu Ala Leu Tyr57 Leu Val Cys Gly Glu Arg Gly Phe Phe Tyr Thr Pro Lys Thr Arg Arg Glu Ala Glu Asp Leu Gln Val Gly Gln Val Glu Leu Gly Gly Gly Pro Gly Ala Gly Ser Leu Gln Pro Leu Ala Leu Glu Gly Ser Leu Gln Lys Arg Gly Ile Val Glu Gln Cys Cys Thr Ser Ile Cys Ser Leu Tyr Gln Leu Glu Asn Tyr Cys Asn | 57 |
| Analog 5 | DNA | ttc gtt aac caa cac ttg tgt ggc tca cac ctg gtg gaa gct ctc tac cta gtg tgc ggg gaa cga gcg ttc ttc tac aca ccc aag acc cgc cgg gag gca gag gac ctg cag gtg ggg cag gtg gag ctg ggc ggg ggc cct ggt gca ggc agc ctg cag ccc ttg gcc ctg gag ggg tcc ctg cag aag cgt ggc att gtg gaa caa tgc tgt acc agc atc tgc tcc ctc tac cag ctg gag aac tac tgc aac | 58 |
| | Protein | Phe Val Asn Gln His Leu Cys Gly Ser His Leu Val Glu Ala Leu Tyr Leu Val Cys Gly Glu Arg Ala Phe Phe Tyr Thr Pro Lys Thr Arg Arg | 59 |
| | | Glu Ala Glu Asp Leu Gln Val Gly Gln Val Glu Leu Gly Gly Gly Pro Gly Ala Gly Ser Leu Gln Pro Leu Ala Leu Glu Gly Ser Leu Gln Lys Arg Gly Ile Val Glu Gln Cys Cys Thr Ser Ile Cys Ser Leu Tyr Gln Leu Glu Asn Tyr Cys Asn | |
| Analog 6 | DNA | ttc gtt aac caa cac ttg tgt ggc tca cac ctg gtg gaa gct ctc tac cta60 gtg tgc ggg gaa cga ggc gcg ttc tac aca ccc aag acc cgc cgg gag gca gag gac ctg cag gtg ggg cag gtg gag ctg ggc ggg ggc cct ggt gca   ggc agc ctg cag ccc ttg gcc ctg gag ggg tcc ctg cag aag cgt ggc att gtg gaa caa tgc tgt acc agc atc tgc tcc ctc tac cag ctg gag aac tac tgc aac | 60 |
| | Protein | Phe Val Asn Gln His Leu Cys Gly Ser His Leu Val Glu Ala Leu Tyr61 Leu Val Cys Gly Glu Arg Gly Ala Phe Tyr Thr Pro Lys Thr Arg Arg Glu Ala Glu Asp Leu Gln Val Gly Gln Val Glu Leu Gly Gly Gly Pro Gly Ala Gly Ser Leu Gln Pro Leu Ala Leu Glu Gly Ser Leu Gln Lys Arg Gly Ile Val Glu Gln Cys Cys Thr Ser Ile Cys Ser Leu Tyr Gln | 61 |
| | | Leu Glu Asn Tyr Cys Asn | |
| Analog 7 | DNA | ttc gtt aac caa cac ttg tgt ggc tca cac ctg gtg gaa gct ctc tac cta 62 gtg tgc ggg gaa cga ggc ttc gcg tac aca ccc aag acc cgc cgg gag gca gag gac ctg cag gtg ggg cag gtg gag ctg ggc ggg ggc cct ggt gca ggc agc ctg cag ccc ttg gcc ctg gag ggg tcc ctg cag aag cgt ggc att gtg gaa caa tgc tgt acc agc atc tgc tcc ctc tac cag ctg gag aac tac tgc aac | 62 |
| | Protein | Phe Val Asn Gln His Leu Cys Gly Ser His Leu Val Glu Ala Leu Tyr 63 Leu Val Cys Gly Glu Arg Gly Phe Ala Tyr Thr Pro Lys Thr Arg Arg Glu Ala Glu Asp Leu Gln Val Gly Gln Val Glu Leu Gly Gly Gly Pro Gly Ala Gly Ser Leu Gln Pro Leu Ala Leu Glu Gly Ser Leu Gln Lys Arg Gly Ile Val Glu Gln Cys Cys Thr Ser Ile Cys Ser Leu Tyr Gln Leu Glu Asn Tyr Cys Asn | 63 |
| Analog 8 | DNA | ttc gtt aac caa cac ttg tgt ggc tca cac ctg gtg gaa gct ctc tac cta64 gtg tgc ggg gaa cga ggc ttc ttc tac aca ccc aag acc cgc cgg gag | 64 |
| | | gca gag gac ctg cag gtg ggg cag gtg gag ctg ggc ggg ggc cct ggt gca ggc agc ctg cag ccc ttg gcc ctg gag ggg tcc ctg cag aag cgt ggc att gtg gaa caa tgc tgt acc agc atc tgc tcc ctc gaa cag ctg gag aac tac tgc aac tga | |
| | Protein | Phe Val Asn Gln His Leu Cys Gly Ser His Leu Val Glu Ala Leu Tyr65 Leu Val Cys Gly Glu Arg Gly Phe Phe Tyr Thr Pro Lys Thr Arg Arg Glu Ala Glu Asp Leu Gln Val Gly Gln Val Glu Leu Gly Gly Gly Pro Gly Ala Gly Ser Leu Gln Pro Leu Ala Leu Glu Gly Ser Leu Gln Lys Arg Gly Ile Val Glu Gln Cys Cys Thr Ser Ile Cys Ser Leu Glu Gln Leu Glu Asn Tyr Cys Asn | 65 |
| Analog 9 | DNA | | 66 |
| | | aac tac tgc aac tga | |
| | Protein | Phe Val Asn Gln His Leu Cys Gly Ser His Leu Val Glu Ala Leu Tyr Leu Val Cys Gly Glu Arg Gly Phe Phe Tyr Thr Pro Lys Thr Arg Arg Glu Ala Glu Asp Leu Gln Val Gly Gln Val Glu Leu Gly Gly Gly Pro Gly Ala Gly Ser Leu Gln Pro Leu Ala Leu Glu Gly Ser Leu Gln Lys Arg Gly Ile Val Glu Gln Cys Cys Thr Ser Ile Cys Ser Leu Asn Gln Leu Glu Asn Tyr Cys Asn | 67 |

Further, the present inventors prepared insulin analogs of Table 4 below with reference to Korean Patent Publication No. 10-2017-0026284.

**[Table 4]**

| Analog | Amino acid alteration |
|---|---|
| Analog 10 | A¹⁴ Tyr -> Glu + B²⁵ deletion |
| Analog 11 | A¹⁴ Tyr -> Ala + B¹⁶ Tyr -> Glu, B²⁵ deletion |

DNA sequences and protein sequences of each of insulin analogs 10 and 11 are shown in Table 5 below.

**[Table 5]**

| | Sequence | | SEQ ID NO: |
|---|---|---|---|
| Analog 10 | DNA | TTC GTT AAC CAA CAC TTG TGT GGC TCA CAC CTG GTG 68 GAA GCT CTC TAC CTA GTG TGC GGG GAA CGA GGC TTC TAC ACA CCC AAG ACC CGC CGG GAG GCA GAG GAC CTG CAG GTG GGG CAG GTG GAG CTG GGC GGG GGC CCT GGT GCA GGC AGC CTG CAG CCC TTG GCC CTG GAG GGG TCC CTG CAG AAG CGT GGC ATT GTG GAA CAA TGC TGT ACC AGC ATC TGC TCC CTC GAA CAG CTG GAG AAC TAC TGC AAC TGA | 68 |
| | Protein | Phe Val Asn Gln His Leu Cys Gly Ser His Leu Val Glu Ala Leu Tyr 69 Leu Val Cys Gly Glu Arg Gly Phe Tyr Thr Pro Lys Thr Arg Arg Glu Ala Glu Asp Leu Gln Val Gly Gln Val Glu Leu Gly Gly Gly Pro Gly Ala Gly Ser Leu Gln Pro Leu Ala Leu Glu Gly Ser Leu Gln Lys Arg Gly Ile Val Glu Gln Cys Cys Thr Ser Ile Cys Ser Leu Glu Gln Leu | 69 |
| | | Glu Asn Tyr Cys Asn | |
| Analog 11 | DNA | TTC GTT AAC CAA CAC TTG TGT GGC TCA CAC CTG GTG GAA GCT CTC GAG CTA GTG TGC GGG GAA CGA GGC TTC TAC ACA CCC AAG ACC CGC CGG GAG GCA GAG GAC CTG CAG GTG GGG CAG GTG GAG CTG GGC GGG GGC CCT GGT GCA GGC AGC CTG CAG CCC TTG GCC CTG GAG GGG TCC CTG CAG AAG CGT GGC ATT GTG GAA CAA TGC TGT ACC AGC ATC TGC TCC CTC GCC CAG CTG GAG AAC TAC TGC AAC TGA | 70 |
| | Protein | Phe Val Asn Gln His Leu Cys Gly Ser His Leu Val Glu Ala Leu Glu Leu Val Cys Gly Glu Arg Gly Phe Tyr Thr Pro Lys Thr Arg Arg Glu Ala Glu Asp Leu Gln Val Gly Gln Val Glu Leu Gly Gly Gly Pro Gly Ala Gly Ser Leu Gln Pro Leu Ala Leu Glu Gly Ser Leu Gln Lys Arg Gly Ile Val Glu Gln Cys Cys Thr Ser Ile Cys Ser Leu Ala Gln Leu Glu Asn Tyr Cys Asn | 71 |

Additionally, the present inventors prepared insulin analogs of Table 6 below. In detail, forward and reverse oligonucleotides (Table 7) were synthesized in order to prepare insulin analogs in which one amino acid of the A-chain or B-chain was modified using the natural insulin expression vector thereof as a template, and PCR was performed to amplify each of the analog genes.

**[Table 6]**

| Analog | Altered sequence |
|---|---|
| Analog 12 | A ¹⁴Y -> H |
| Analog 13 | A ¹⁴Y -> K |
| Analog 14 | A ¹⁹Y -> E |
| Analog 15 | A ¹⁹Y -> S |
| Analog 16 | A ¹⁹Y -> T |
| Analog 17 | B ¹⁶Y -> E |
| Analog 18 | B ¹⁶Y -> S |
| Analog 19 | B ¹⁶Y -> T |
| Analog 20 | A ¹⁴Y -> A |
| Analog 21 | A ¹⁴Y -> D |
| Analog 22 | B ¹⁶Y -> D |
| Analog 23 | B ²⁵F -> D |
| Analog 24 | B ²⁵F -> E |

Primers for amplification of the insulin analogs are shown in Table 7 below.

**[Table 7]**

| Analog | Sequence | SEQ ID NO |
|---|---|---|
| Analog 12 | 5' CAGCATCTGCTCCCTCCATCAGCTGGAGAACTAC 3' | 72 |
| | 5' GTAGTTCTCCAGCTGATGGAGGGAGCAGARGCTG 3' | 73 |
| Analog 13 | 5' CAGCATCTGCTCCCTCAAGCAGCTGGAGAACTAC 3' | 74 |
| | 5' GTAGTTCTCCAGCTGCTTGAGGGAGCAGATGCTG 3' | 75 |
| Analog 14 | 5' CTACCAGCTGGAGAACGAGTGCAACTGAGGATCC 3' | 76 |
| | 5' GGATCCTCAGTTGCACTCGTTCTCCAGCTGGTAG 3' | 77 |
| Analog 15 | 5' CTACCAGCTGGAGAACTCCTGCAACTGAGGATCC 3' | 78 |
| | 5' GGATCCTCAGTTGCAGGAGTTCTCCAGCTGGTAG 3' | 79 |
| Analog 16 | 5' CTACCAGCTGGAGAACACCTGCAACTGAGGATCC 3' | 80 |
| | 5' GGATCCTCAGTTGCAGGTGTTCTCCAGCTGGTAG 3' | 81 |
| Analog 17 | 5' CTGGTGGAAGCTCTCGAGCTAGTGTGCGGGGAAC 3' | 82 |
| | 5' GTTCCCCGCACACTAGCTCGAGAGCTTCCACCAG 3' | 83 |
| Analog 18 | 5' CTGGTGGAAGCTCTCTCCCTAGTGTGCGGGGAAC 3' | 84 |
| | 5' GTTCCCCGCACACTAGGGAGAGAGCTTCCACCAG 3' | 85 |
| Analog 19 | 5' CTGGTGGAAGCTCTCACCCTAGTGTGCGGGGAAC 3' | 86 |
| | 5' GTTCCCCGCACACTAGGGTGAGAGCTTCCACCAG 3' | 87 |
| Analog 20 | 5' CAGCATCTGCTCCCTCGCCCAGCTGGAGAACTAC 3' | 88 |
| | 5' GTAGTTCTCCAGCTGGGCGAGGGAGCAGATGCTG 3' | 89 |
| Analog 21 | 5' CAGCATCTGCTCCCTCGACCAGCTGGAGAACTAC 3' | 90 |
| | 5' GTAGTTCTCCAGCTGGTCGAGGGAGCAGATGCTG 3' | 91 |
| Analog 22 | 5' CTGGTGGAAGCTCTCGACCTAGTGTGCGGGGAAC 3' | 92 |
| | 5' GTTCCCCGCACACTAGGTCGAGAGCTTCCACCAG 3' | 93 |
| Analog 23 | 5' GGGGAACGAGGCTTCGACTACACACCCAAGACC 3' | 94 |
| | 5' GGTCTTGGGTGTGTAGTCGAAGCCTCGTTCCCC 3' | 95 |
| Analog 24 | 5' GGGGAACGAGGCTTCGAGTACACACCCAAGACC 3' | 96 |
| | 5' GGTCTTGGGTGTGTACTCGAAGCCTCGTTCCCC 3' | 97 |

For amplification of the insulin analogs, PCR was performed under conditions of 18 cycles at 95°C for 30 sec, 55°C for 30 sec, and 68°C 6 min. The insulin analog fragments obtained under the above conditions were inserted into a pET22b vector for expression in the form of an intracellular inclusion body, and the obtained expression vectors were named as pET22b-insulin analogs 1 to 13. The expression vectors included nucleotides encoding amino acid sequences of each of insulin analogs 1 to 13 under the control of T7 promoter. Each insulin analog protein was expressed in the form of an inclusion body in the host cells including the expression vector.

DNA sequences and protein sequences of each of insulin analogs 1 to 13 are shown in Table 4 below.

Each alteration in the sequences was examined by DNA sequencing analysis, and as a result, it was confirmed that the sequence alteration occurred in each insulin analog according to the purpose.

**[Table 8]**

| nalog | Sequence | | SEQ ID NO |
|---|---|---|---|
| Analog 12 | DNA | TTC GTT AAC CAA CAC TTG TGT GGC TCA 98 CAC CTG GTG GAA GCT CTC TAC CTA GTG TGC GGG GAA CGA GGC TTC TTC TAC ACA CCC AAG ACC CGC CGG GAG GCA GAG GAC CTG CAG GTG GGG CAG GTG GAG CTG GGC GGG GGC CCT GGT GCA GGC AGC CTG CAG CCC TTG GCC CTG GAG GGG TCC CTG CAG AAG CGT GGC ATT GTG GAA CAA TGC TGT ACC AGC ATC TGC TCC CTC CAT CAG CTG GAG AAC TAC TGC AAC | 98 |
| | Protein | Phe Val Asn Gln His Leu Cys Gly Ser His Leu Val 99 Glu Ala Leu Tyr Leu Val Cys Gly Glu Arg Gly Phe Phe Tyr Thr Pro Lys Thr Arg Arg Glu Ala Glu Asp Leu Gln Val Gly Gln Val Glu Leu Gly Gly Gly Pro | 99 |
| | | Gly Ala Gly Ser Leu Gln Pro Leu Ala Leu Glu Gly Ser Leu Gln Lys Arg Gly Ile Val Glu Gln Cys Cys Thr Ser Ile Cys Ser Leu His Gln Leu Glu Asn Tyr Cys Asn | |
| Analog 13 | DNA | TTC GTT AAC CAA CAC TTG TGT GGC TCA 100 CAC CTG GTG GAA GCT CTC TAC CTA GTG TGC GGG GAA CGA GGC TTC TTC TAC ACA CCC AAG ACC CGC CGG GAG GCA GAG GAC CTG CAG GTG GGG CAG GTG GAG CTG GGC GGG GGC CCT GGT GCA GGC AGC CTG CAG CCC TTG GCC CTG GAG GGG TCC CTG CAG AAG CGT GGC ATT GTG GAA CAA TGC TGT ACC AGC ATC TGC TCC CTC AAG CAG CTG GAG AAC TAC TGC AAC | 100 |
| | Protein | Phe Val Asn Gln His Leu Cys Gly Ser His Leu Val 101 | 101 |
| | | Glu Ala Leu Tyr Leu Val Cys Gly Glu Arg Gly Phe Phe Tyr Thr Pro Lys Thr Arg Arg Glu Ala Glu Asp Leu Gln Val Gly Gln Val Glu Leu Gly Gly Gly Pro Gly Ala Gly Ser Leu Gln Pro Leu Ala Leu Glu Gly Ser Leu Gln Lys Arg Gly Ile Val Glu Gln Cys Cys Thr Ser Ile Cys Ser Leu Lys Gln Leu Glu Asn Tyr Cys Asn | |
| Analog 14 | DNA | TTC GTT AAC CAA CAC TTG TGT GGC TCA 102 CAC CTG GTG GAA GCT CTC TAC CTA GTG TGC GGG GAA CGA GGC TTC TTC TAC ACA CCC AAG ACC CGC CGG GAG GCA GAG GAC CTG CAG GTG GGG CAG GTG GAG CTG GGC GGG GGC CCT GGT GCA GGC AGC CTG CAG CCC TTG GCC CTG GAG GGG TCC CTG CAG AAG CGT GGC ATT GTG GAA CAA TGC TGT | 102 |
| | | ACC AGC ATC TGC TCC CTC TAC CAG CTG GAG AAC GAG TGC AAC | |
| | Protein | Phe Val Asn Gln His Leu Cys Gly Ser His Leu Val Glu Ala Leu Tyr Leu Val Cys Gly Glu Arg Gly Phe Phe Tyr Thr Pro Lys Thr Arg Arg Glu Ala Glu Asp Leu Gln Val Gly Gln Val Glu Leu Gly Gly Gly Pro Gly Ala Gly Ser Leu Gln Pro Leu Ala Leu Glu Gly Ser Leu Gln Lys Arg Gly Ile Val Glu Gln Cys Cys Thr Ser Ile Cys Ser Leu Tyr Gln Leu Glu Asn Glu Cys Asn | 103 |
| Analog 15 | DNA | TTC GTT AAC CAA CAC TTG TGT GGC TCA CAC CTG GTG GAA GCT CTC TAC CTA GTG TGC GGG GAA CGA GGC TTC TTC TAC ACA CCC AAG ACC CGC CGG GAG GCA GAG GAC CTG CAG GTG GGG CAG GTG GAG CTG GGC | 104 |
| | | GGG GGC CCT GGT GCA GGC AGC CTG CAG CCC TTG GCC CTG GAG GGG TCC CTG CAG AAG CGT GGC ATT GTG GAA CAA TGC TGT ACC AGC ATC TGC TCC CTC TAC CAG CTG GAG AAC TCC TGC AAC | |
| | Protein | Phe Val Asn Gln His Leu Cys Gly Ser His Leu Val 105 Glu Ala Leu Tyr Leu Val Cys Gly Glu Arg Gly Phe Phe Tyr Thr Pro Lys Thr Arg Arg Glu Ala Glu Asp Leu Gln Val Gly Gln Val Glu Leu Gly Gly Gly Pro Gly Ala Gly Ser Leu Gln Pro Leu Ala Leu Glu Gly Ser Leu Gln Lys Arg Gly Ile Val Glu Gln Cys Cys Thr Ser Ile Cys Ser Leu Tyr Gln Leu Glu Asn Ser Cys Asn | 105 |
| Analog 16 | DNA | TTC GTT AAC CAA CAC TTG TGT GGC TCA 106 CAC CTG GTG GAA GCT CTC TAC CTA GTG | 105 |
| | | TGC GGG GAA CGA GGC TTC TTC TAC ACA CCC AAG ACC CGC CGG GAG GCA GAG GAC CTG CAG GTG GGG CAG GTG GAG CTG GGC GGG GGC CCT GGT GCA GGC AGC CTG CAG CCC TTG GCC CTG GAG GGG TCC CTG CAG AAG CGT GGC ATT GTG GAA CAA TGC TGT ACC AGC ATC TGC TCC CTC TAC CAG CTG GAG AAC ACC TGC AAC | |
| | Protein | Phe Val Asn Gln His Leu Cys Gly Ser His Leu Val 107 Glu Ala Leu Tyr Leu Val Cys Gly Glu Arg Gly Phe Phe Tyr Thr Pro Lys Thr Arg Arg Glu Ala Glu Asp Leu Gln Val Gly Gln Val Glu Leu Gly Gly Gly Pro Gly Ala Gly Ser Leu Gln Pro Leu Ala Leu Glu Gly Ser Leu Gln Lys Arg Gly Ile Val Glu Gln Cys Cys Thr Ser Ile Cys Ser Leu Tyr Gln Leu Glu Asn Thr | 107 |
| | | Cys Asn | |
| Analog 17 | DNA | TTC GTT AAC CAA CAC TTG TGT GGC TCA 108 CAC CTG GTG GAA GCT CTC GAG CTA GTG TGC GGG GAA CGA GGC TTC TTC TAC ACA CCC AAG ACC CGC CGG GAG GCA GAG GAC CTG CAG GTG GGG CAG GTG GAG CTG GGC GGG GGC CCT GGT GCA GGC AGC CTG CAG CCC TTG GCC CTG GAG GGG TCC CTG CAG AAG CGT GGC ATT GTG GAA CAA TGC TGT ACC AGC ATC TGC TCC CTC TAC CAG CTG GAG AAC TAC TGC AAC | 108 |
| | Protein | Phe Val Asn Gln His Leu Cys Gly Ser His Leu Val 109 Glu Ala Leu Glu Leu Val Cys Gly Glu Arg Gly Phe Phe Tyr Thr Pro Lys Thr Arg Arg Glu Ala Glu Asp Leu Gln Val Gly Gln Val Glu Leu Gly Gly Gly Pro | 109 |
| | | Gly Ala Gly Ser Leu Gln Pro Leu Ala Leu Glu Gly Ser Leu Gln Lys Arg Gly Ile Val Glu Gln Cys Cys Thr Ser Ile Cys Ser Leu Tyr Gln Leu Glu Asn Tyr Cys Asn | |
| Analog 18 | DNA | TTC GTT AAC CAA CAC TTG TGT GGC TCA 110 CAC CTG GTG GAA GCT CTC TCC CTA GTG TGC GGG GAA CGA GGC TTC TTC TAC ACA CCC AAG ACC CGC CGG GAG GCA GAG GAC CTG CAG GTG GGG CAG GTG GAG CTG GGC GGG GGC CCT GGT GCA GGC AGC CTG CAG CCC TTG GCC CTG GAG GGG TCC CTG CAG AAG CGT GGC ATT GTG GAA CAA TGC TGT ACC AGC ATC TGC TCC CTC TAC CAG CTG GAG AAC TAC TGC AAC | 110 |
| | Protein | Phe Val Asn Gln His Leu Cys Gly Ser His Leu Val 111 | 111 |
| | | Glu Ala Leu Ser Leu Val Cys Gly Glu Arg Gly Phe Phe Tyr Thr Pro Lys Thr Arg Arg Glu Ala Glu Asp Leu Gln Val Gly Gln Val Glu Leu Gly Gly Gly Pro Gly Ala Gly Ser Leu Gln Pro Leu Ala Leu Glu Gly Ser Leu Gln Lys Arg Gly Ile Val Glu Gln Cys Cys Thr Ser Ile Cys Ser Leu Tyr Gln Leu Glu Asn Tyr Cys Asn | |
| Analog 19 | DNA | TTC GTT AAC CAA CAC TTG TGT GGC TCA 112 CAC CTG GTG GAA GCT CTC ACC CTA GTG TGC GGG GAA CGA GGC TTC TTC TAC ACA CCC AAG ACC CGC CGG GAG GCA GAG GAC CTG CAG GTG GGG CAG GTG GAG CTG GGC GGG GGC CCT GGT GCA GGC AGC CTG CAG CCC TTG GCC CTG GAG GGG TCC CTG CAG AAG CGT GGC ATT GTG GAA CAA TGC TGT | 112 |
| | | ACC AGC ATC TGC TCC CTC TAC CAG CTG GAG AAC TAC TGC AAC | |
| | Protein | Phe Val Asn Gln His Leu Cys Gly Ser His Leu Val 113 Glu Ala Leu Thr Leu Val Cys Gly Glu Arg Gly Phe Phe Tyr Thr Pro Lys Thr Arg Arg Glu Ala Glu Asp Leu Gln Val Gly Gln Val Glu Leu Gly Gly Gly Pro Gly Ala Gly Ser Leu Gln Pro Leu Ala Leu Glu Gly Ser Leu Gln Lys Arg Gly Ile Val Glu Gln Cys Cys Thr Ser Ile Cys Ser Leu Tyr Gln Leu Glu Asn Tyr Cys Asn | 113 |
| Analog 20 | DNA | TTC GTT AAC CAA CAC TTG TGT GGC TCA 114 CAC CTG GTG GAA GCT CTC TAC CTA GTG TGC GGG GAA CGA GGC TTC TTC TAC ACA CCC AAG ACC CGC CGG GAG GCA GAG GAC CTG CAG GTG GGG CAG GTG GAG CTG GGC | 114 |
| | | GGG GGC CCT GGT GCA GGC AGC CTG CAG CCC TTG GCC CTG GAG GGG TCC CTG CAG AAG CGT GGC ATT GTG GAA CAA TGC TGT ACC AGC ATC TGC TCC CTC GCC CAG CTG GAG AAC TAC TGC AAC | |
| | Protein | Phe Val Asn Gln His Leu Cys Gly Ser His Leu Val 115 Glu Ala Leu Tyr Leu Val Cys Gly Glu Arg Gly Phe Phe Tyr Thr Pro Lys Thr Arg Arg Glu Ala Glu Asp Leu Gln Val Gly Gln Val Glu Leu Gly Gly Gly Pro Gly Ala Gly Ser Leu Gln Pro Leu Ala Leu Glu Gly Ser Leu Gln Lys Arg Gly Ile Val Glu Gln Cys Cys Thr Ser Ile Cys Ser Leu Ala Gln Leu Glu Asn Tyr Cys Asn | 115 |
| Analog 21 | DNA | TTC GTT AAC CAA CAC TTG TGT GGC TCA 116 CAC CTG GTG GAA GCT CTC TAC CTA GTG | 116 |
| | | TGC GGG GAA CGA GGC TTC TTC TAC ACA CCC AAG ACC CGC CGG GAG GCA GAG GAC CTG CAG GTG GGG CAG GTG GAG CTG GGC GGG GGC CCT GGT GCA GGC AGC CTG CAG CCC TTG GCC CTG GAG GGG TCC CTG CAG AAG CGT GGC ATT GTG GAA CAA TGC TGT ACC AGC ATC TGC TCC CTC GAC CAG CTG GAG AAC TAC TGC AAC | |
| | Protein | Phe Val Asn Gln His Leu Cys Gly Ser His Leu Val Glu Ala Leu Tyr Leu Val Cys Gly Glu Arg Gly Phe Phe Tyr Thr Pro Lys Thr Arg Arg Glu Ala Glu Asp Leu Gln Val Gly Gln Val Glu Leu Gly Gly Gly Pro Gly Ala Gly Ser Leu Gln Pro Leu Ala Leu Glu Gly Ser Leu Gln Lys Arg Gly Ile Val Glu Gln Cys Cys Thr Ser Ile Cys Ser Leu Asp Gln Leu Glu Asn Tyr | 117 |
| | | Cys Asn | |
| Analog 22 | DNA | TTC GTT AAC CAA CAC TTG TGT GGC TCA CAC CTG GTG GAA GCT CTC GAC CTA GTG TGC GGG GAA CGA GGC TTC TTC TAC ACA CCC AAG ACC CGC CGG GAG GCA GAG GAC CTG CAG GTG GGG CAG GTG GAG CTG GGC GGG GGC CCT GGT GCA GGC AGC CTG CAG CCC TTG GCC CTG GAG GGG TCC CTG CAG AAG CGT GGC ATT GTG GAA CAA TGC TGT ACC AGC ATC TGC TCC CTC TAC CAG CTG GAG AAC TAC TGC AAC | 118 |
| | Protein | Phe Val Asn Gln His Leu Cys Gly Ser His Leu Val Glu Ala Leu Asp Leu Val Cys Gly Glu Arg Gly Phe Phe Tyr Thr Pro Lys Thr Arg Arg Glu Ala Glu Asp Leu Gln Val Gly Gln Val Glu Leu Gly Gly Gly Pro | 119 |
| | | Gly Ala Gly Ser Leu Gln Pro Leu Ala Leu Glu Gly Ser Leu Gln Lys Arg Gly Ile Val Glu Gln Cys Cys Thr Ser Ile Cys Ser Leu Tyr Gln Leu Glu Asn Tyr Cys Asn | |
| Analog 23 | DNA | TTC GTT AAC CAA CAC TTG TGT GGC TCA 120 CAC CTG GTG GAA GCT CTC TAC CTA GTG TGC GGG GAA CGA GGC TTC GAC TAC ACA CCC AAG ACC CGC CGG GAG GCA GAG GAC CTG CAG GTG GGG CAG GTG GAG CTG GGC GGG GGC CCT GGT GCA GGC AGC CTG CAG CCC TTG GCC CTG GAG GGG TCC CTG CAG AAG CGT GGC ATT GTG GAA CAA TGC TGT ACC AGC ATC TGC TCC CTC TAC CAG CTG GAG AAC TAC TGC AAC | 120 |
| | Protein | Phe Val Asn Gln His Leu Cys Gly Ser His Leu Val 121 | 121 |
| | | Glu Ala Leu Tyr Leu Val Cys Gly Glu Arg Gly Phe Asp Tyr Thr Pro Lys Thr Arg Arg Glu Ala Glu Asp Leu Gln Val Gly Gln Val Glu Leu Gly Gly Gly Pro Gly Ala Gly Ser Leu Gln Pro Leu Ala Leu Glu Gly Ser Leu Gln Lys Arg Gly Ile Val Glu Gln Cys Cys Thr Ser Ile Cys Ser Leu Tyr Gln Leu Glu Asn Tyr Cys Asn | |
| Analog 24 | DNA | TTC GTT AAC CAA CAC TTG TGT GGC TCA 122 CAC CTG GTG GAA GCT CTC TAC CTA GTG TGC GGG GAA CGA GGC TTC GAG TAC ACA CCC AAG ACC CGC CGG GAG GCA GAG GAC CTG CAG GTG GGG CAG GTG GAG CTG GGC GGG GGC CCT GGT GCA GGC AGC CTG CAG CCC TTG GCC CTG GAG GGG TCC CTG CAG AAG CGT GGC ATT GTG GAA CAA TGC TGT | 122 |
| | | ACC AGC ATC TGC TCC CTC TAC CAG CTG GAG AAC TAC TGC AAC | |
| | Protein | Phe Val Asn Gln His Leu Cys Gly Ser His Leu Val Glu Ala Leu Tyr Leu Val Cys Gly Glu Arg Gly Phe Glu Tyr Thr Pro Lys Thr Arg Arg Glu Ala Glu Asp Leu Gln Val Gly Gln Val Glu Leu Gly Gly Gly Pro Gly Ala Gly Ser Leu Gln Pro Leu Ala Leu Glu Gly Ser Leu Gln Lys Arg Gly Ile Val Glu Gln Cys Cys Thr Ser Ile Cys Ser Leu Tyr Gln Leu Glu Asn Tyr Cys Asn | 123 |

### Example 3: Expression of Recombinant Insulin Analog Fusion Peptide

Expression of the recombinant insulin analogs was performed under the control of T7 promoter. *E. coli* BL21-DE3 (*E*. *coli* B F-dcm ompT hsdS(rB-mB-) gal λDE3); Novagen) was transformed with each recombinant insulin analog expression vector. The transformation method was performed according to a method recommended by Novagen. Each of single colonies transformed with each recombinant expression vector was taken, inoculated into 2X Luria Broth (LB) medium containing ampicillin (50 µg/mL), and incubated at 37°C for 15 hours. The culture broth of recombinant strain and 2X LB medium containing 30% glycerol were mixed at a ratio of 1:1 (v/v), and each 1 mL thereof was dispensed into cryo-tubes and stored at -140°C, and then used as a cell stock for the production of recombinant fusion proteins.

For expression of the recombinant insulin analogs, 1 vial of each cell stock was thawed and inoculated in 500 mL of 2X Luria Broth (LB) medium, and then incubated with shaking at 37°C for 14 hours to 16 hours. When the OD₆₀₀ value reached 5.0 or more, the culturing was terminated, and this culture broth was used as a seed culture. The seed culture was inoculated into 17 L of a fermentation medium using a 50 L fermentor (MSJ-U2, B.E. MARUBISHI, Japan), and initial bath fermentation was begun. The culture conditions were maintained at a temperature of 37°C, an air flow rate of 20 L/min (1 vvm), an agitation speed of 500 rpm, and pH 6.70 by using a 30% ammonia solution. Fermentation was carried out in a fed-batch mode by adding a feeding solution, when nutrients were depleted in the culture broth. Growth of the strain was monitored by OD value. IPTG was introduced at a final concentration of 500 µM when the OD value was above 100. After introduction, the culturing was further carried out for about 23 hours to 25 hours. After terminating the culturing, the recombinant strains were harvested using a centrifuge and stored at -80°C until use.

### Example 4: Recovery and Refolding of Recombinant Insulin Analogs

In order to change the recombinant insulin analogs expressed in Example 3 into soluble forms, cells were disrupted, followed by refolding. 100 g (wet weight) of the cell pellet was re-suspended in 1 L of lysis buffer (50 mM Tris-HCI (pH 9.0), 1 mM EDTA (pH 8.0), 0.2 M NaCl and 0.5% Triton X-100). The cells were disrupted using a microfluidizer processor (Microfluidic Corp. Model M-110EH-30) at an operating pressure of 15,000 psi. The cell lysate thus disrupted was centrifuged at 7,000 rpm at 4°C to 8°C for 20 minutes. The supernatant was discarded, and the pellet was re-suspended in 3 L of washing buffer (0.5% Triton X-100 and 50 mM Tris-HCI (pH 8.0), 0.2 M NaCl, 1 mM EDTA). After centrifugation at 7,000 rpm at 4°C to 8°C for 20 minutes, the cell pellet was re-suspended in distilled water, followed by centrifugation in the same manner. The pellet was taken and re-suspended in a buffer (1 M L-glycine, 3.78 g L-cysteine-HCl, pH 10.6), and stirred at room temperature for 1 hour. To recover the recombinant insulin analog thus re-suspended, 8 M urea was added, followed by stirring for 3 hours to 5 hours. For refolding of the solubilized recombinant insulin analogs, centrifugation was carried out at 7,000 rpm at 4°C to 8°C for 30 minutes, and the supernatant was obtained and treated with a reducing agent (15 mM L-cysteine-HCl) for 1 hour. A predetermined multiple-equivalent of distilled water was added thereto using a peristaltic pump while stirring at 4°C to 8°C for 12 hours or more.

### Example 5: Cation-Exchange Chromatography Purification

The refolded sample was loaded onto an SP FF (GE Healthcare) column equilibrated with 20 mM sodium citrate (pH 2.0) buffer containing 45% ethanol, and then the insulin analog proteins were eluted in 10 column volumes with a linear gradient from 0% to 100% using 20 mM sodium citrate (pH 2.0) buffer containing 0.5 M potassium chloride and 45% ethanol.

### Example 6: Trypsin and Carboxypeptidase B Treatment

Salts were removed from the eluted samples using an ultrafiltration membrane, and the buffer was exchanged with a buffer (10 mM Tris-HCl, pH 8.0). With respect to the obtained sample protein, trypsin corresponding to a molar ratio of about 30,000 and carboxypeptidase B corresponding to a molar ratio of about 30,000 were added, and then this was stirred at 4°C to 8°C for 16 hours or more.

### Example 7: Cation-Exchange Chromatography Purification

The sample thus reacted was loaded onto an SP HP (GE Healthcare) column equilibrated with 20 mM sodium citrate (pH 2.0) buffer containing 45% ethanol, and then the insulin analog proteins were eluted in 10 column volumes with a linear gradient from 0% to 100% using 20 mM sodium citrate (pH 2.0) buffer containing 0.5 M potassium chloride and 45% ethanol.

### Example 8: Reversed-Phase Chromatography Purification

To isolate pure insulin analog from the sample obtained in Example 7, the sample was loaded onto a reversed-phase chromatography Source30RPC (GE Healthcare, USA) equilibrated with a buffer containing sodium phosphate and isopropanol, and then the insulin analog proteins were eluted with a linear gradient using a buffer containing sodium phosphate and isopropanol.

### Example 9: Preparation of Long-Acting Conjugates of Insulin and Glucagon

Long-acting conjugates of insulin and glucagon were prepared by linking the insulin and the glucagon analog prepared in the Examples and an immunoglobulin Fc region using polyethylene glycol as a linker, respectively. The glucagon analog used in the preparation of the long-acting glucagon conjugate was an analog having a pl of 6 to 7 and *in vitro* activity of 200% or more relative to that of the native glucagon. The insulin used in the preparation of the long-acting insulin conjugate was a natural insulin or an analog obtained by substituting one amino acid of the A-chain of natural insulin.

In detail, for pegylation of the N-terminus of the B-chain of insulin (Biocon, India) with 3.4 K propion-ALD(2) PEG (3.4 kDa PEG having one propionaldehyde group each at both ends, NOF, USA), the insulin and PEG were reacted at an insulin:PEG molar ratio of 1:4 with an insulin concentration of 5 mg/mL at 4°C for about 2 hours. At this time, the reaction was performed in a mixed solvent of 50 mM sodium citrate (pH 5.0) and 45% isopropanol while adding 3 mM sodium cyanoborohydride (NaCNBH₃) as a reducing agent. The reaction solution was purified with an SP-HP (GE Healthcare) column using a buffer containing sodium citrate (pH 3.0) and 45% ethanol and a KCI concentration gradient.

Next, to link insulin-PEG to the N-terminus of an immunoglobulin Fc fragment, the purified mono-PEGylated insulin and the immunoglobulin Fc fragment were reacted at a molar ratio of 1:1.2 at 25°C for 15 hours, with a total protein concentration of about 20 mg/mL. At this time, to the reaction solution, 100 mM HEPES buffer (pH 8.2) and sodium chloride, and 20 mM sodium cyanoborohydride as a reducing agent were added.

After the reaction was terminated, the reaction solution was loaded onto a Q HP (GE, USA) column with Tris-HCI (pH 7.5) buffer and a NaCl concentration gradient, and loaded onto a Source 15ISO (GE, USA) with an ammonium sulfate and Tris-HCI (pH 7.5) concentration gradient to purify an insulin-3.4K PEG-immunoglobulin Fc conjugate.

Further, to prepare a long-acting glucagon conjugate, for PEGylation of a cysteine residue of the glucagon analog with 10 kDa PEG having a maleimide group and an aldehyde group each at both ends, i.e., maleimide-PEG-aldehyde (10 kDa, NOF, Japan), the reaction was carried out with a molar ratio of glucagon analog to maleimide-PEG-aldehyde of 1:1 to 5 and a peptide concentration of 3 mg/mL to 10 mg/mL for 1 hour to 3 hours at low temperature. At this time, the reaction was carried out in an environment in which 50 mM Tris buffer (pH 7.5) and 20% to 60% isopropanol were added. After the reaction was terminated, the reaction solution was loaded onto SP sepharose HP (GE Healthcare, USA) to purify a glucagon derivative mono-pegylated to cysteine.

Next, the mono-pegylated glucagon analog thus purified and the immunoglobulin Fc were reacted at a molar ratio of 1:2 to 10 and a protein concentration of 10 mg/mL to 50 mg/mL at 4°C to 8°C for 12 hours to 18 hours. The reaction was carried out in an environment in which 10 mM to 50 mM sodium cyanoborohydride as a reducing agent and 10% to 20% isopropanol were added to 100 mM potassium phosphate buffer (pH 6.0). After the reaction was terminated, the reaction solution was loaded onto a butyl sepharose FF purification column (GE Healthcare, USA) and a Source ISO purification column (GE Healthcare, USA) to purify a conjugate including the glucagon analog and the immunoglobulin Fc.

### Example 10: Examination of Blood Glucose-Lowering Effect of Combined Administration of Glucagon and Insulin in DIO/STZ Rats

The long-acting conjugates of glucagon and natural insulin prepared in Example 9 were administered to DIO/STZ rats, which are known as an obesity and type 2 diabetes model. To examine the blood glucose-lowering effect of the combined administration of the long-acting conjugates of glucagon and natural insulin, test groups were divided into a vehicle control, single administration of the long-acting insulin conjugate (53.9 nmol/kg (2997 µg/kg), Q3D), and combined administration of the long-acting insulin conjugate (53.9 nmol/kg, Q3D) and the long-acting glucagon conjugate (3.4 nmol/kg (182 µg/kg), Q3D), and each of the corresponding materials was repeatedly administered subcutaneously for 2 weeks. During the repeated administration for 2 weeks, each rat's blood was taken from the tail vein, and then changes in the blood glucose were measured using a blood glucose meter (OneTouch® Ultra®).

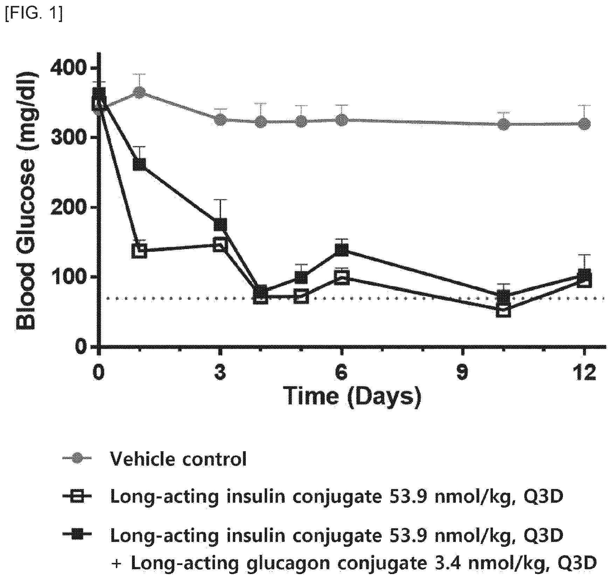

As a result of the repeated administration of the long-acting insulin conjugate for 2 weeks, the high blood glucose levels observed in the vehicle control dropped to normal and hypoglycemic levels (70 mg/dL or less) (FIG. 1). These results indicate that high doses of insulin exhibited a blood glucose-lowering effect, but could induce hypoglycemia. In contrast, when combined administration of the long-acting insulin conjugate and the long-acting glucagon conjugate was performed, blood glucose was normalized without persistent hypoglycemia.

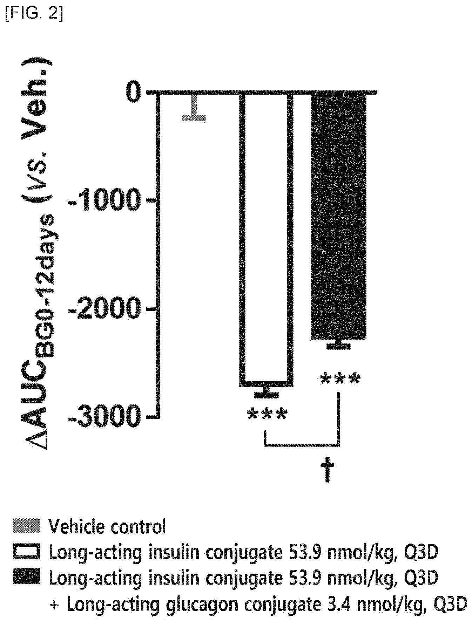

Further, changes in the blood glucose during the administration period were quantified by area under the curve (AUC) and analyzed as shown in FIG. 2. As a result, when combined administration of the long-acting insulin conjugate and the long-acting glucagon conjugate was performed, the combined administration of the long-acting insulin conjugate and the long-acting glucagon conjugate did not significantly affect the overall blood glucose-lowering effect of the long-acting insulin conjugate while preventing hypoglycemia, which is a side effect caused by administration of the long-acting insulin conjugate (FIG. 2). These results indicate that the combined administration of the long-acting glucagon conjugate and the long-acting insulin conjugate may maintain the blood glucose-lowering efficacy while reducing the risk of hypoglycemia of the long-acting insulin conjugate.

### Example 11: Effect of Improving Side Effects of Insulin by Combined Administration of Glucagon and Insulin in DIO/STZ Rats

The long-acting conjugate of natural insulin used in Example 10 also has a side effect of weight gain along with hypoglycemia, like other long-acting insulins.

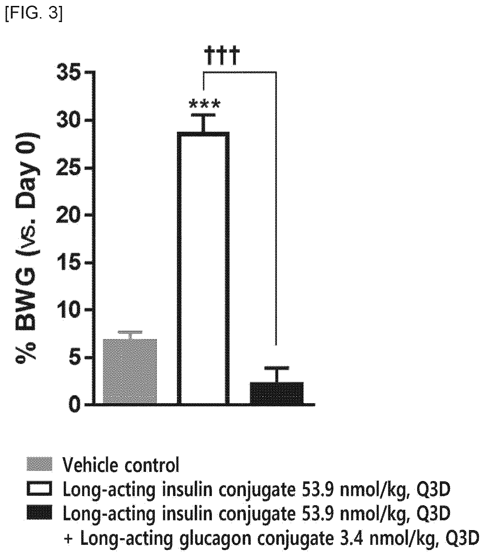

In fact, as shown in FIG. 3, when the long-acting insulin conjugate was repeatedly administered to DIO/STZ rats for 2 weeks, a significant increase in body weight was observed, as compared with the vehicle control. However, when combined administration of the long-acting glucagon conjugate and the long-acting insulin conjugate was performed, the weight gain was neutralized, and the weight loss was observed, as compared with the vehicle control. These results indicate that combined administration of the long-acting insulin conjugate and the long-acting glucagon conjugate may alleviate hypoglycemia and weight gain, which are side effects caused by administration of the long-acting insulin conjugate.

The above exemplary embodiments confirmed that combined administration of the long-acting insulin conjugate and the long-acting glucagon conjugate may suppress the side effects (hypoglycemia and weight gain) caused by insulin administration while obtaining a blood glucose-lowering effect. Thus, it was confirmed that the combined administration of the long-acting insulin conjugate and the long-acting glucagon conjugate according to the present invention may not only have a therapeutic effect on insulin-related diseases, but may also improve hypoglycemia and insulin side effects.

Based on the above description, it will be understood by those skilled in the art that the present disclosure may be implemented in a different specific form without changing the technical spirit or essential characteristics thereof. In this regard, it should be understood that the above embodiment is not limitative, but illustrative in all aspects. The scope of the disclosure is defined by the appended claims rather than by the description preceding them, and therefore all changes and modifications that fall within metes and bounds of the claims, or equivalents of such metes and bounds, are therefore intended to be embraced by the claims.

## Claims

1. A pharmaceutical composition for preventing or treating an insulin-related disease, the pharmaceutical composition comprising insulin and glucagon.

2. The pharmaceutical composition of claim 1, wherein the insulin is linked to a biocompatible material capable of increasing *in vivo* half-life thereof to be in the form of a long-acting conjugate.

3. The pharmaceutical composition of claim 1, wherein the glucagon is linked to a biocompatible material capable of increasing *in vivo* half-life thereof to be in the form of a long-acting conjugate.

4. The pharmaceutical composition of claim 1, wherein the insulin is linked to a biocompatible material capable of increasing *in vivo* half-life thereof to be in the form of a long-acting conjugate, and the glucagon is linked to a biocompatible material capable of increasing *in vivo* half-life thereof to be in the form of a long-acting conjugate.

5. The pharmaceutical composition of any one of claims 1 to 4, wherein the insulin-related disease is selected from the group consisting of an insulin-resistant disease, diabetes, hyperglycemia, and obesity.

6. The pharmaceutical composition of any one of claims 1 to 4, wherein the composition alleviates hypoglycemia, which is a side effect of insulin, and suppresses weight gain.

7. The pharmaceutical composition of any one of claims 1 to 4, wherein the composition includes the insulin or the long-acting conjugate thereof and the glucagon or the long-acting conjugate thereof at a weight ratio of 0.1:1 to 100:1.

8. The pharmaceutical composition of any one of claims 1 to 4, wherein the glucagon is a native glucagon or a glucagon analog obtained by alteration selected from the group consisting of substitution, addition, deletion, modification, and a combination thereof in one or more amino acids of the native glucagon.

9. The pharmaceutical composition of claim 8, wherein the glucagon analog includes an amino acid sequence of the following General Formula 1: 
X1–X2–QGTF–X7–SD–X10–S–X12–X13–X14–X15–X16–X17–X18–X19–
X20–X21–F–X23–X24–W–L–X27–X28–T–X30 (General Formula 1, SEQ ID
NO: 46)
 wherein in General Formula 1,
X1 is histidine (H), desamino-histidyl, dimethyl-histidyl (*N*-dimethyl-histidyl), *beta*-hydroxy imidazopropionyl, 4-imidazoacetyl, *beta*-carboxy imidazopropionyl, tryptophan (W), or tyrosine (Y), or is absent;
X2 is *alpha*-methyl-glutamic acid (a-methyl-glutamic acid), Aib (aminoisobutyric acid), D-alanine, glycine (G), Sar (*N*-methylglycine), serine (S), or D-serine;
X7 is threonine (T), valine (V), or cysteine (C);
X10 is tyrosine (Y) or cysteine (C);
X12 is lysine (K) or cysteine (C);
X13 is tyrosine (Y) or cysteine (C);
X14 is leucine (L) or cysteine (C);
X15 is aspartic acid (D), glutamic acid (E), or cysteine (C);
X16 is glutamic acid (E), aspartic acid (D), serine (S), *alpha*-methyl-glutamic acid, or cysteine (C), or is absent;
X17 is aspartic acid (D), glutamine (Q), glutamic acid (E), lysine (K), arginine (R), serine (S), cysteine (C), or valine (V), or is absent;
X18 is alanine (A), aspartic acid (D), glutamine (Q), glutamic acid (E), arginine (R), valine (V), or cysteine (C), or is absent;
X19 is alanine (A), arginine (R), serine (S), valine (V), or cysteine (C), or is absent;
X20 is lysine (K), histidine (H), glutamic acid (E), glutamine (Q), aspartic acid (D), arginine (R), *alpha*-methyl-glutamic acid, or cysteine (C), or is absent;
X21 is aspartic acid (D), glutamic acid (E), leucine (L), valine (V), or cysteine (C), or is absent;
X23 is isoleucine (I), valine (V), or arginine (R), or is absent;
X24 is valine (V), arginine (R), alanine (A), cysteine (C), glutamic acid (E), lysine (K), glutamine (Q), *alpha*-methyl-glutamic acid, or leucine (L), or is absent;
X27 is isoleucine (I), valine (V), alanine (A), lysine (K), methionine (M), glutamine (Q), or arginine (R), or is absent;
X28 is glutamine (Q), lysine (K), asparagine (N), or arginine (R), or is absent; and
X30 is cysteine (C), or is absent (with the proviso that when the amino acid sequence of General Formula 1 is the same as SEQ ID NO: 1, it is excluded).

10. The pharmaceutical composition of claim 9, wherein in General Formula 1,
X1 is histidine (H), tryptophan (W), or tyrosine (Y), or is absent;
X2 is serine (S) or Aib (aminoisobutyric acid);
X7 is threonine (T), valine (V), or cysteine (C);
X10 is tyrosine (Y) or cysteine (C);
X12 is lysine (K) or cysteine (C);
X13 is tyrosine (Y) or cysteine (C);
X14 is leucine (L) or cysteine (C);
X15 is aspartic acid (D) or cysteine (C);
X16 is glutamic acid (E), serine (S), or cysteine (C);
X17 is aspartic acid (D), glutamic acid (E), lysine (K), arginine (R), serine (S), cysteine (C), or valine (V);
X18 is aspartic acid (D), glutamic acid (E), arginine (R), or cysteine (C);
X19 is alanine (A) or cysteine (C);
X20 is glutamine (Q), aspartic acid (D), lysine (K), or cysteine (C);
X21 is aspartic acid (D), glutamic acid (E), leucine (L), valine (V), or cysteine (C);
X23 is isoleucine (I), valine (V), or arginine (R);
X24 is valine (V), arginine (R), alanine (A), glutamic acid (E), lysine (K), glutamine (Q), or leucine (L);
X27 is isoleucine (I), valine (V), alanine (A), methionine (M), glutamine (Q), or arginine (R);
X28 is glutamine (Q), lysine (K), asparagine (N), or arginine (R); and
X30 is cysteine (C) or is absent.

11. The pharmaceutical composition of claim 9, wherein in General Formula 1,
X1 is histidine (H), tryptophan (W), or tyrosine (Y);
X2 is serine (S) or Aib (aminoisobutyric acid);
X7 is cysteine (C), threonine (T), or valine (V);
X10 is tyrosine (Y) or cysteine (C);
X12 is lysine (K) or cysteine (C);
X13 is tyrosine (Y) or cysteine (C);
X14 is leucine (L) or cysteine (C);
X15 is aspartic acid (D) or cysteine (C);
X16 is glutamic acid (E), serine (S), or cysteine (C);
X17 is glutamic acid (E), lysine (K), arginine (R), cysteine (C), or valine (V);
X18 is arginine (R) or cysteine (C);
X19 is alanine (A) or cysteine (C);
X20 is glutamine (Q) or lysine (K);
X21 is aspartic acid (D), glutamic acid (E), valine (V), or cysteine (C);
X23 is valine (V);
X24 is valine (V) or glutamine (Q);
X27 is methionine (M);
X28 is asparagine (N) or arginine (R); and
X30 is cysteine (C) or is absent.

12. The pharmaceutical composition of claim 9, wherein in General Formula 1,
X1 is tyrosine (Y);
X2 is Aib (aminoisobutyric acid);
X7 is cysteine (C), threonine (T), or valine (V);
X10 is tyrosine (Y) or cysteine (C);
X12 is lysine (K);
X13 is tyrosine (Y) or cysteine (C);
X14 is leucine (L) or cysteine (C);
X15 is aspartic acid (D) or cysteine (C);
X16 is glutamic acid (E), serine (S), or cysteine (C);
X17 is lysine (K), arginine (R), cysteine (C), or valine (V);
X18 is arginine (R) or cysteine (C);
X19 is alanine (A) or cysteine (C);
X20 is glutamine (Q) or lysine (K);
X21 is aspartic acid (D), glutamic acid (E), or cysteine (C);
X23 is valine (V);
X24 is glutamine (Q);
X27 is methionine (M);
X28 is asparagine (N) or arginine (R); and
X30 is cysteine (C) or is absent.

13. The pharmaceutical composition of claim 9, wherein in General Formula 1,
X1 is histidine (H), tryptophan (W), or tyrosine (Y), or is absent;
X2 is serine (S) or Aib (aminoisobutyric acid);
X7 is threonine (T), valine (V), or cysteine (C);
X10 is tyrosine (Y) or cysteine (C);
X12 is lysine (K) or cysteine (C);
X13 is tyrosine (Y) or cysteine (C);
X14 is leucine (L) or cysteine (C);
X15 is aspartic acid (D), or cysteine (C);
X16 is glutamic acid (E), serine (S), or cysteine (C);
X17 is aspartic acid (D), glutamic acid (E), lysine (K), arginine (R), serine (S), cysteine (C), or valine (V);
X18 is aspartic acid (D), glutamic acid (E), arginine (R), or cysteine (C);
X19 is alanine (A), or cysteine (C);
X20 is glutamine (Q), aspartic acid (D), or lysine (K);
X21 is aspartic acid (D), or glutamic acid (E);
X23 is valine (V);
X24 is valine (V) or glutamine (Q);
X27 is isoleucine (I) or methionine (M);
X28 is asparagine (N) or arginine (R); and
X30 is cysteine (C) or is absent.

14. The pharmaceutical composition of claim 9, wherein in General Formula 1,
X1 is tyrosine (Y);
X2 is Aib (aminoisobutyric acid);
X7 is threonine (T);
X10 is tyrosine (Y);
X12 is lysine (K);
X13 is tyrosine (Y);
X14 is leucine (L);
X15 is aspartic acid (D) or cysteine (C);
X16 is glutamic acid (E), serine (S), or cysteine (C);
X17 is lysine (K) or arginine (R);
X18 is arginine (R);
X19 is alanine (A);
X20 is glutamine (Q), cysteine (C), or lysine (K);
X21 is aspartic acid (D), cysteine (C), valine (V), or glutamic acid (E);
X23 is valine (V) or arginine (R);
X24 is glutamine (Q) or leucine (L);
X27 is methionine (M);
X28 is asparagine (N) or arginine (R); and
X30 is absent.

15. The pharmaceutical composition of claim 9, wherein the glucagon analog includes an amino acid sequence of the following General Formula 2: 
Y–Aib–QGTF–X7–SD–X10–S–X12–Y–L–X15–X16–X17–R–A–X20–X21–
F–V–X24–W–L–M–N–T–X30 (General Formula 2, SEQ ID NO: 47)
 wherein in General Formula 2,
X7 is threonine (T), valine (V), or cysteine (C);
X10 is tyrosine (Y) or cysteine (C);
X12 is lysine (K) or cysteine (C);
X15 is aspartic acid (D) or cysteine (C);
X16 is glutamic acid (E) or serine (S);
X17 is lysine (K) or arginine (R);
X20 is glutamine (Q) or lysine (K);
X21 is aspartic acid (D) or glutamic acid (E);
X24 is valine (V) or glutamine (Q); and
X30 is cysteine (C) or is absent.

16. The pharmaceutical composition of claim 9, wherein one or more amino acid pairs among the amino acid pairs of X10 and X14, X12 and X16, X16 and X20, X17 and X21, X20 and X24, and X24 and X28 in General Formula 1 each form a ring between respective amino acids.

17. The pharmaceutical composition of claim 16, wherein each amino acid of one or more amino acid pairs among the amino acid pairs of X10 and X14, X12 and X16, X16 and X20, X17 and X21, X20 and X24, and X24 and X28 in General Formula 1 is substituted with glutamic acid or lysine, which is capable of forming a ring.

18. The pharmaceutical composition of claim 9, wherein the glucagon analog includes an amino acid sequence selected from the group consisting of SEQ ID NOS: 2 to 45.

19. The pharmaceutical composition of claim 18, wherein the glucagon analog includes an amino acid sequence of SEQ ID NO: 37.

20. The pharmaceutical composition of any one of claims 1 to 4, wherein the insulin is a native insulin or an insulin analog obtained by alteration selected from the group consisting of substitution, addition, deletion, modification, and a combination thereof in one or more amino acids of the native insulin.

21. The pharmaceutical composition of claim 20, wherein the insulin analog is an insulin analog obtained by substituting another amino acid for, or by deleting one or more amino acids selected from the group consisting of, an amino acid at position 1, an amino acid at position 2, an amino acid at position 3, an amino acid at position 5, an amino acid at position 8, an amino acid at position 10, an amino acid at position 12, an amino acid at position 16, an amino acid at position 23, an amino acid at position 24, an amino acid at position 25, an amino acid at position 26, an amino acid at position 27, an amino acid at position 28, an amino acid at position 29, and an amino acid at position 30 of a B-chain of the natural insulin, and an amino acid at position 1, an amino acid at position 2, an amino acid at position 5, an amino acid at position 8, an amino acid at position 10, an amino acid at position 12, an amino acid at position 14, an amino acid at position 16, an amino acid at position 17, an amino acid at position 18, an amino acid at position 19, and an amino acid at position 21 of an A-chain of the natural insulin.

22. The pharmaceutical composition of claim 20, wherein the insulin analog includes an A-chain of SEQ ID NO: 48 represented by the following General Formula 3 and a B-chain of SEQ ID NO: 49 represented by the following General Formula 4: 
[General Formula 3]
Xaa1–Xaa2–Val–Glu–Xaa5–Cys–Cys–Thr–Ser–Ile–Cys–Xaa12–Leu–
Xaa14–Gln–Xaa16–Glu–Asn–Xaa19–Cys–Xaa21 (SEQ ID NO: 48)
 wherein in General Formula 3,
Xaa1 is alanine, glycine, glutamine, histidine, glutamic acid, or asparagine;
Xaa2 is alanine or isoleucine;
Xaa5 is alanine, glutamic acid, glutamine, histidine, or asparagine;
Xaa12 is alanine, serine, glutamine, glutamic acid, histidine, or asparagine;
Xaa14 is alanine, tyrosine, glutamic acid, histidine, lysine, aspartic acid, or asparagine;
Xaa16 is alanine, leucine, tyrosine, histidine, glutamic acid, or asparagine;
Xaa19 is alanine, tyrosine, serine, glutamic acid, histidine, threonine, or asparagine; and
Xaa21 is asparagine, glycine, histidine, or alanine; 
[General Formula 4]
Phe–Val–Asn–Gln–His–Leu–Cys–Xaa8–Ser–His–Leu–Val–Glu–Ala–Leu–
Xaa16–Leu–Val–Cys–Gly–Glu–Arg–Xaa23–Xaa24–Xaa25–Tyr–Xaa27–Xaa28–
Lys–Thr (SEQ ID NO: 49)
 wherein in General Formula 4,
Xaa8 is alanine or glycine;
Xaa16 is tyrosine, glutamic acid, serine, threonine, or aspartic acid, or is absent;
Xaa23 is glycine or alanine;
Xaa24 is alanine or phenylalanine;
Xaa25 is alanine, phenylalanine, aspartic acid, or glutamic acid, or is absent;
Xaa27 is threonine or is absent; and
Xaa28 is proline, glutamic acid, or aspartic acid, or is absent (with the proviso that when a peptide includes an A-chain of SEQ ID NO: 48 and a B-chain of SEQ ID NO: 49, it is excluded).

23. The pharmaceutical composition of claim 22, wherein the insulin analog is obtained by substituting alanine for one or more amino acids selected from the group consisting of an amino acid at position 8, an amino acid at position 23, an amino acid at position 24, and an amino acid at position 25 of the B-chain of the natural insulin, and an amino acid at position 1, an amino acid at position 2, and an amino acid at position 19 of the A-chain of the natural insulin, or by substituting glutamic acid or asparagine for an amino acid at position 14 of the A-chain of the natural insulin.

24. The pharmaceutical composition of claim 23, wherein the insulin analog includes an amino acid sequence selected from the group consisting of SEQ ID NOS: 51, 53, 55, 57, 59, 61, 63, 65, and 67.

25. The pharmaceutical composition of claim 22, wherein the insulin analog is obtained by substituting glutamic acid for an amino acid at position 16 of the B-chain of the natural insulin; by deleting an amino acid at position 25 of the B-chain of the natural insulin; or by substituting glutamic acid or alanine for an amino acid at position 14 of the A-chain of the natural insulin.

26. The pharmaceutical composition of claim 25, wherein the insulin analog includes an amino acid sequence of SEQ ID NO: 69 or 71.

27. The pharmaceutical composition of claim 22, wherein the insulin analog is obtained by substituting glutamic acid, serine, threonine, or aspartic acid for an amino acid at position 16 of the B-chain of the natural insulin; by substituting aspartic acid or glutamic acid for an amino acid at position 25 of the B-chain of the natural insulin; by substituting histidine, lysine, alanine, or aspartic acid for an amino acid at position 14 of the A-chain of the natural insulin; or by substituting glutamic acid, serine, or threonine for an amino acid at position 19 of the A-chain of the natural insulin.

28. The pharmaceutical composition of claim 27, wherein the insulin analog includes an amino acid sequence selected from the group consisting of SEQ ID NOS: 99, 101, 103, 105, 107, 109, 111, 113, 115, 117, 119, 121, and 123.

29. The pharmaceutical composition of claim 22, wherein the insulin analog is in the form of two polypeptide chains consisting of the A-chain of SEQ ID NO: 48 represented by General Formula 3 and the B-chain of SEQ ID NO: 49 represented by General Formula 4.

30. The pharmaceutical composition of claim 29, wherein the A-chain and the B-chain are linked to each other via a disulfide bond.

31. The pharmaceutical composition of any one of claims 2 to 4, wherein the conjugate is represented by the following Chemical Formula 1:
[Chemical Formula 1] X-La-F
wherein in Chemical Formula 1,
X is insulin or glucagon;
L is a linker;
a is 0 or a natural number, provided that when a is 2 or more, each L is independent;
F is a material capable of increasing *in vivo* half-life of X; and
"-" represents a covalent or non-covalent bond.

32. The pharmaceutical composition of claim 31, wherein F is selected from the group consisting of a high-molecular-weight polymer, a fatty acid, cholesterol, albumin and a fragment thereof, an albumin-binding material, a polymer of repeating units of a particular amino acid sequence, an antibody, an antibody fragment, an FcRn-binding material, an *in vivo* connective tissue, a nucleotide, fibronectin, transferrin, a saccharide, heparin, and elastin.

33. The pharmaceutical composition of claim 32, wherein the high-molecular-weight polymer is selected from the group consisting of polyethylene glycol, polypropylene glycol, an ethylene glycol-propylene glycol copolymer, polyoxyethylated polyol, polyvinyl alcohol, a polysaccharide, polyvinyl ethyl ether, a biodegradable polymer, a lipid polymer, chitin, hyaluronic acid, an oligonucleotide, and a combination thereof.

34. The pharmaceutical composition of claim 31, wherein F is an immunoglobulin Fc region.

35. The pharmaceutical composition of claim 34, wherein F is an IgG Fc region.

36. The pharmaceutical composition of claim 34, wherein the immunoglobulin Fc region is aglycosylated.

37. The pharmaceutical composition of claim 34, wherein the immunoglobulin Fc region is selected from the group consisting of (a) a CH1 domain, a CH2 domain, a CH3 domain, and a CH4 domain; (b) a CH1 domain and a CH2 domain; (c) a CH1 domain and a CH3 domain; (d) a CH2 domain and a CH3 domain; (e) a combination between one or two or more domains among a CH1 domain, a CH2 domain, a CH3 domain, and a CH4 domain and an immunoglobulin hinge region or a part of the hinge region; and (f) a dimer between each domain of the heavy chain constant region and the light chain constant region.

38. The pharmaceutical composition of claim 34, wherein the immunoglobulin Fc region is an immunoglobulin Fc region, in which the region capable of forming a disulfide bond is deleted, in which a part of the amino acid(s) in the N-terminus of native Fc is deleted, in which a methionine residue is added to the N-terminus of native Fc, in which a complement-binding site is deleted, or in which an antibody-dependent cell-mediated cytotoxicity (ADCC) site is deleted.

39. The pharmaceutical composition of claim 34, wherein the immunoglobulin Fc region is an immunoglobulin Fc fragment derived from IgG, IgA, IgD, IgE, or IgM.

40. The pharmaceutical composition of claim 34, wherein the immunoglobulin Fc region is a hybrid of a domain having a different origin derived from an immunoglobulin selected from the group consisting of IgG, IgA, IgD, IgE, and IgM.

41. The pharmaceutical composition of claim 31, wherein L is selected from the group consisting of a peptide, a fatty acid, saccharide, a high-molecular-weight polymer, a low-molecular-weight compound, a nucleotide, and a combination thereof.

42. The pharmaceutical composition of claim 41, wherein the high-molecular-weight polymer is selected from the group consisting of polyethylene glycol, polypropylene glycol, an ethylene glycol-propylene glycol copolymer, polyoxyethylated polyol, polyvinyl alcohol, a polysaccharide, polyvinyl ethyl ether, a biodegradable polymer, a lipid polymer, chitin, hyaluronic acid, an oligonucleotide, and a combination thereof.

43. The pharmaceutical composition of claim 31, wherein L is polyethylene glycol.

44. The pharmaceutical composition of claim 1, wherein the insulin and the glucagon are administered in combination simultaneously, sequentially, or in reverse order.

45. A pharmaceutical composition for preventing or treating hypoglycemia, the pharmaceutical composition comprising insulin and glucagon.

46. The pharmaceutical composition of claim 45, wherein the insulin is linked to a biocompatible material capable of increasing *in vivo* half-life thereof to be in the form of a long-acting conjugate.

47. The pharmaceutical composition of claim 45, wherein the glucagon is linked to a biocompatible material capable of increasing *in vivo* half-life thereof to be in the form of a long-acting conjugate.

48. The pharmaceutical composition of claim 45, wherein the insulin is linked to a biocompatible material capable of increasing *in vivo* half-life thereof to be in the form of a long-acting conjugate, and the glucagon is linked to a biocompatible material capable of increasing *in vivo* half-life thereof to be in the form of a long-acting conjugate.

49. The pharmaceutical composition of claim 45, wherein the insulin and the glucagon are administered in combination simultaneously, sequentially, or in reverse order.

50. A composition for improving side effects of insulin, the composition comprising insulin and glucagon.

51. The composition of claim 50, wherein the insulin is linked to a biocompatible material capable of increasing *in vivo* half-life thereof to be in the form of a long-acting conjugate.

52. The composition of claim 50, wherein the glucagon is linked to a biocompatible material capable of increasing *in vivo* half-life thereof to be in the form of a long-acting conjugate.

53. The composition of claim 50, wherein the insulin is linked to a biocompatible material capable of increasing *in vivo* half-life thereof to be in the form of a long-acting conjugate, and the glucagon is linked to a biocompatible material capable of increasing *in vivo* half-life thereof to be in the form of a long-acting conjugate.

54. The composition of claim 50, wherein the insulin and the glucagon are administered in combination simultaneously, sequentially, or in reverse order.

55. A complex formulation for improving hypoglycemia of a patient with an insulin-related disease, the complex formulation comprising insulin and glucagon.

56. The complex formulation of claim 55, wherein the insulin is linked to a biocompatible material capable of increasing *in vivo* half-life thereof to be in the form of a long-acting conjugate.

57. The complex formulation of claim 55, wherein the glucagon is linked to a biocompatible material capable of increasing *in vivo* half-life thereof to be in the form of a long-acting conjugate.

58. The complex formulation of claim 55, wherein the insulin is linked to a biocompatible material capable of increasing *in vivo* half-life thereof to be in the form of a long-acting conjugate, and the glucagon is linked to a biocompatible material capable of increasing *in vivo* half-life thereof to be in the form of a long-acting conjugate.

59. The complex formulation of any one of claims 55 to 58, wherein the hypoglycemia is a side effect of insulin.

60. The complex formulation of any one of claims 55 to 58, wherein the complex formulation suppresses weight gain.

61. The complex formulation of any one of claims 55 to 58, wherein the complex formulation includes the insulin or the long-acting conjugate thereof and the glucagon or the long-acting conjugate thereof at a weight ratio of 0.1:1 to 100:1.
